# EUROPEAN PATENT APPLICATION

(11) **EP 2 101 173 A1**
(43) Date of publication of application: **16.09.2009**
(21) Application number: 08300146.1
(22) Date of filing: 14.03.2008
(51) Int. Cl.: G01N 33/50, G01N 33/576, G01N 33/68

(54) **In vitro method to determine whether a drug candidate active against a target protein is active against a variant of said protein**

(71) Applicant: Vivalis, 49450 Roussay (FR)
(72) Inventor: Lahmar, Medhi, 44100 Nantes (FR); Valarche, Isabelle, 44220 Coueron (FR); Mehtali, Majid, 44220 Coueron (FR)
(74) Representative: Warcoin, Jacques

(57) **Abstract**

The present invention relates to the field of virology. More precisely, the invention provides a method of determining the ability of a test compound to modulate the biological activity of a variant of a target protein, wherein said test compound is previously known to modulate the biological activity of said protein. This invention is useful to determine whether a drug candidate, such as anti-viral compounds (e.g. against hepatitis C virus: NS5B, NS3), active against a target protein is active against a variant of said protein (e.g. polymorphisms, genotypes or mutants).

SEQUENCE LISTING

<110>  Vivalis

<120>  In vitro method to determine whether a drug candidate active
       against a target protein is active against a variant of said
       protein

<130>  D26388

<160>  61

<170>  PatentIn version 3.3

<210>  1
<211>  1779
<212>  DNA
<213>  Hepatitis C Virus


<220>
<223>  genotype 1b polymerase NS5B

<400>  1
atgtcaatgt cctacacatg gacaggtgcc ttgatcacgc catgcgctgc ggaggagagc      60

aagttgccca tcaatccgtt gagcaactct ttgctgcgtc accacagtat ggtctactcc     120

acaacatctc gcagcgcaag tctgcggcag aagaaggtaa cctttgacag aatgcaagtc     180

ctggacgacc actaccggga cgtgctcaag gagatgaagg cgaaggcgtc cacagttaag     240

gctaggcttc tatctataga ggaggcctgc aaactgacgc ccccacattc ggccaaatcc     300

aaatttggct acggggcgaa ggacgtccgg agcctatcca gcagggccgt caaccacatc     360

cgctccgtgt gggaggactt gctggaagac actgaaacac caattgatac caccatcatg     420

gcaaaaaatg aggttttctg cgtccaacca gagaaaggag gccgcaagcc agctcgcctt     480

atcgtattcc cagacctggg ggtacgtgta tgcgagaaga tggcccttta cgacgtggtc     540

tccacccttc ctcaggccgt gatgggcccc tcatacggat tccagtactc tcctgggcag     600

cgggtcgagt tcctggtgaa tacctggaaa tcaaagaaat gccctatggg cttctcatat     660

gacacccgct gctttgactc aacggtcact gagaatgaca tccgtactga ggagtcaatc     720

taccaatgtt gtgacttggc ccccgaagcc agacaggcca taaagtcgct cacagagcgg     780

ctctacatcg ggggtcccct gactaattca aaagggcaga actgcggtta tcgccggtgc     840

cgcgcgagcg gtgtgctgac gactagctgc ggcaataccc tcacatgcta cttgaaagcc     900

actgcggcct gtcgagctgc aaagctccag gactgcacga tgctcgtgaa cggagacgac     960

cttgtcgtta tctgcgaaag cgcgggaacc caggaggatg cggcgagcct acgagtcttc    1020

acggaggcta tgactaggta ctctgccccc cccggggacc cgccccaacc agaatacgac    1080

ttggagctga taacgtcatg ctcctccaat gtgtcggtcg cgcacgatgc atccggcaaa    1140

agggtgtact acctcacccg tgaccccacc acccccctcg cacgggctgc gtgggagaca 1200

gttagacaca ctccagtcaa ctcctggcta ggcaatatca tcatgtatgc gcccacccta 1260

tgggcgagga tgattctgat gactcatttc ttctctatcc ttctagctca ggagcaactt 1320

gaaaaagccc tggattgtca gatctacggg gcctgctact ccattgagcc acttgaccta 1380

cctcagatca tcgaacgact ccatggtctt agcgcatttt cactccatag ttactctcca 1440

ggtgagatca atagggtggc ttcatgcctc aggaaacttg gggtaccgcc cttgcgagtc 1500

tggagacatc gggccagaag tgtccgcgct aagctgctgt cccagggggg gagggccgcc 1560

acatgcggca aatacctctt caactgggca gtaaggacca agcttaaact cactccaatc 1620

ccggctgcgt cccagctaga cttgtccggc tggttcgttg ctggttacaa cgggggagac 1680

atatatcaca gcctgtctcg tgcccgaccc cgttggttca tgttgtgcct actcctactt 1740

tctgtagggg taggcatcta cctgctcccc aaccggtaa 1779

<210> 2
<211> 1890
<212> DNA
<213> Hepatitis C Virus

<220>
<223> genotype 1b NS3 full length protein

<400> 2
atcacggcct attcccaaca aacgcggggc ctgcttggct gtatcatcac tagcctcaca 60

ggtcgggaca agaaccaggt cgatggggag gttcaggtgc tctccaccgc aacgcaatct 120

ttcctggcga cctgcgtcaa tggcgtgtgt tggaccgtct accatggtgc cggctcgaag 180

accctggccg gcccgaaggg tccaatcacc caaatgtaca ccaatgtaga ccaggacctc 240

gtcggctggc cggcgccccc cggggcgcgc tccatgacac cgtgcacctg cggcagctcg 300

gacctttact tggtcacgag gcatgccgat gtcattccgg tgcgccggcg aggcgacagc 360

agggggagtc tactctcccc taggcccgtc tcctacctga agggctcctc gggtggacca 420

ctgctttgcc cttcggggca cgttgtaggc atcttccggg ctgctgtgtg cacccggggg 480

gttgcgaagg cggtggactt catacccgtt gagtctatgg aaactaccat gcggtctccg 540

gtcttcacag acaactcatc ccctccggcc gtaccgcaaa cattccaagt ggcacattta 600

cacgctccca ctggcagcgg caagagcacc aaagtgccgg ctgcatatgc agcccaaggg 660

tacaaggtgc tcgtcctaaa cccgtccgtt gctgccacat tgggctttgg agcgtatatg 720

tccaaggcac atggcatcga gcctaacatc agaactgggg taaggaccat caccacgggc 780

ggccccatca cgtactccac ctatggcaag ttccttgccg acggtggatg ctccgggggc 840

gcctatgaca tcataatatg tgacgaatgc cactcaactg actggacaac catcttgggc 900

atcggcacag tcctggatca ggcagagacg gctggagcgc ggctcgtcgt gctcgccacc 960

gccacgcctc cgggatcgat caccgtgcca caccccaaca tcgaggaagt ggccctgtcc 1020

aacactgggg agattccctt ctatggcaaa gccatcccca ttgaggccat caagggggga 1080

aggcatctca tcttctgcca ttccaagaag aagtgtgacg agctcgccgc aaagctgaca 1140

ggcctcggac tcaatgctgt agcgtattac cggggtctcg atgtgtccgt cataccgact 1200

agcggagacg tcgttgtcgt ggcaacagac gctctaatga cgggctttac cggcgacttt 1260

gactcagtga tcgactgcaa cacatgtgtc acccagacag tcgatttcag cttggacccc 1320

accttcacca ttgagacgac aaccgtgccc caagacgcgg tgtcgcgctc gcagcggcga 1380

ggtaggactg gcaggggcag gagtggcatc tacaggtttg tgactccagg agaacggccc 1440

tcaggcatgt tcgactcctc ggtcctgtgt gagtgctatg acgcaggctg cgcttggtat 1500

gagctcacgc ccgctgagac tacagtcagg ttgcgggctt acctgaatac accagggttg 1560

cccgtctgcc aggaccatct ggagttctgg gaaagcgtct tcacaggcct cacccacata 1620

gatgcccact tcctgtccca aaccaagcag gcaggagaca acttccccta cctggtggca 1680

taccaagcca cggtgtgcgc cagggctcag gctccacctc catcgtggga tcaaatgtgg 1740

aagtgtctca tacggcttaa acctacgctg cacgggccaa cacccctgct gtataggcta 1800

ggagccgttc aaaatgagat caccctcaca catcccataa ccaaattcgt catggcatgc 1860

atgtcggccg acctggaggt cgtcacttaa 1890

<210> 3
<211> 34
<212> DNA
<213> Artificial

<220>
<223> Forward primer

<400> 3
tatctcgagt caatgtccta cacatggaca ggtg 34

<210> 4
<211> 30
<212> DNA
<213> Artificial

<220>
<223> Reverse Primer

<400> 4
agatctgcag ttaacggggt cgggcacgag 30

<210>  5
<211>  84
<212>  DNA
<213>  Artificial

<220>
<223>  Forward primer

<400>  5
tactcgaggg ttctgttgtt attgttggta gaattatttt atctggtagt ggtagtatca     60

cggcctattc ccaacaaacg cggg                                            84


<210>  6
<211>  32
<212>  DNA
<213>  Artificial

<220>
<223>  Reverse primer

<400>  6
acctcgagca gcgcctatca cggcctattc cc                                   32


<210>  7
<211>  84
<212>  DNA
<213>  Artificial

<220>
<223>  Forward primer

<400>  7
tactcgaggg ttctgttgtt attgttggta gaattatttt atctggtagt ggtagtatca     60

cggcctattc ccaacaaacg cggg                                            84


<210>  8
<211>  28
<212>  DNA
<213>  Artificial

<220>
<223>  Reverse primer

<400>  8
atagatcttt taagtgacga cctccagg                                        28


<210>  9
<211>  29
<212>  DNA
<213>  Artificial

<220>
<223>  Forward Primer

<400>  9
ttcctcgagg atcaatgtcc tacacatgg                                          29


<210>  10
<211>  30
<212>  DNA
<213>  Artificial

<220>
<223>  Reverse Primer

<400>  10
gctcggatcc cagctctcaa cggggtcggg                                         30


<210>  11
<211>  39
<212>  DNA
<213>  Artificial

<220>
<223>  Forward Primer

<400>  11
aatccgttga gcaactcttc gctgcgtcac cacagtatg                               39


<210>  12
<211>  38
<212>  DNA
<213>  Artificial

<220>
<223>  Reverse Primer

<400>  12
atactgtggt gacgcagcga agagttgctc aacggatt                                38


<210>  13
<211>  39
<212>  DNA
<213>  Artificial

<220>
<223>  Forward Primer

<400>  61
aatccgttga gcaactctcg gctgcgtcac cacagtatg                               39

<210>  14
<211>  39
<212>  DNA
<213>  Artificial

<220>
<223>  Reverse Primer

<400>  14
catactgtgg tgacgcagcc gagagttgct caacggatt                               39

<210>  15
<211>  27
<212>  DNA
<213>  Artificial

<220>
<223>  Forward primer

<400>  15
ctgacgcccc cacgctcggc caaatcc                                          27


<210>  16
<211>  27
<212>  DNA
<213>  Artificial

<220>
<223>  Reverse Primer

<400>  16
ctgacgcccc cacgctcggc caaatcc                                          27


<210>  17
<211>  27
<212>  DNA
<213>  Artificial

<220>
<223>  Forward primer

<400>  17
ggcaatatca tcacctatgc gcccacc                                          27


<210>  18
<211>  27
<212>  DNA
<213>  Artificial

<220>
<223>  Reverse Primer

<400>  18
ggcaatatca tcacctatgc gcccacc                                          27


<210>  19
<211>  27
<212>  DNA
<213>  Artificial

<220>
<223>  Forward Primer

<400>  19
ggcaatatca tcacctatgc gcccacc                                          27

<210>  20
<211>  27
<212>  DNA
<213>  Artificial

<220>
<223>  Reverse Primer

<400>  20
ggcaatatca tcacctatgc gcccacc                                          27


<210>  21
<211>  27
<212>  DNA
<213>  Artificial

<220>
<223>  Forward Primer

<400>  21
ctatgggcga ggacgattct gatgact                                          27


<210>  22
<211>  27
<212>  DNA
<213>  Artificial

<220>
<223>  Reverse Primer

<400>  22
agtcatcaga atcgtcctcg cccatag                                          27


<210>  23
<211>  26
<212>  DNA
<213>  Artificial

<220>
<223>  Forward Primer

<400>  23
atcatggcaa aaactgaggt tttctg                                           26


<210>  24
<211>  26
<212>  DNA
<213>  Artificial

<220>
<223>  Reverse Primer

<400>  24
cagaaaacct cagtttttgc catgat                                           26

<210>  25
<211>  27
<212>  DNA
<213>  Artificial

<220>
<223>  Forward primer

<400>  25
gaggccgcaa gctagctcgc cttatcg                                          27


<210>  26
<211>  27
<212>  DNA
<213>  Artificial

<220>
<223>  Reverse Primer

<400>  26
cgataaggcg agctagcttg cggcctc                                          27


<210>  27
<211>  27
<212>  DNA
<213>  Artificial

<220>
<223>  Forward Primer

<400>  27
ggaaacttgg ggtactaccc ttgcgag                                          27


<210>  28
<211>  27
<212>  DNA
<213>  Artificial

<220>
<223>  Reverse Primer

<400>  28
ctcgcaaggg tagtacccca agtttcc                                          27


<210>  29
<211>  24
<212>  DNA
<213>  Artificial

<220>
<223>  Forward Primer

<400>  29
ggtgccgcgc gaccggcgtg ctga                                             24


<210>  30

<211>  24
<212>  DNA
<213>  Artificial

<220>
<223>  Reverse Primer

<400>  30
tcagcacgcc ggtcgcgcgg cacc                                              24


<210>  31
<211>  22
<212>  DNA
<213>  Artificial

<220>
<223>  Forward primer

<400>  31
gtcatgctcc gccaatgtgt cg                                                22


<210>  32
<211>  22
<212>  DNA
<213>  Artificial

<220>
<223>  Reverse Primer

<400>  32
cgacacattg gcggagcatg ac                                                22


<210>  33
<211>  26
<212>  DNA
<213>  Artificial

<220>
<223>  Forward Primer

<400>  33
atgcgcccac catgtgggcg aggatg                                            26


<210>  34
<211>  26
<212>  DNA
<213>  Artificial

<220>
<223>  Reverse Primer

<400>  34
catcctcgcc cacatggtgg gcgcat                                            26


<210>  35
<211>  27

<212>  DNA
<213>  Artificial

<220>
<223>  Forward primer

<400>  35
acgcccccac atacggccaa atccaaa                                          27


<210>  36
<211>  27
<212>  DNA
<213>  Artificial

<220>
<223>  Reverse Primer

<400>  36
tttggatttg gccgtatgtg ggggcgt                                          27


<210>  37
<211>  24
<212>  DNA
<213>  Artificial

<220>
<223>  Forward Primer

<400>  37
gtagcaggcc cccatgatct gaca                                             24


<210>  38
<211>  24
<212>  DNA
<213>  Artificial

<220>
<223>  Reverse Primer

<400>  38
gtagcaggcc cccatgatct gaca                                             24


<210>  39
<211>  86
<212>  DNA
<213>  Artificial

<220>
<223>  Forward Primer

<400>  39
tactcgagca ggttctgttg ttattgttgg tagaattatt ttatctggta gtggtagtat      60

cacggcctat tcccaacaaa cgcggg                                           86


<210>  40

<211>  32
<212>  DNA
<213>  Artificial

<220>
<223>  Reverse Primer

<400>  40
acctcgagca gcgcctatca cggcctattc cc                                    32


<210>  41
<211>  23
<212>  DNA
<213>  Artificial

<220>
<223>  Forward primer

<400>  41
ggcatcttcc gggttgctgt gtg                                              23


<210>  42
<211>  23
<212>  DNA
<213>  Artificial

<220>
<223>  Reverse Primer

<400>  42
cacacagcaa cccggaagat gcc                                              23


<210>  43
<211>  86
<212>  DNA
<213>  Artificial

<220>
<223>  Forward primer

<400>  43
tactcgagca ggttctgttg ttattgttgg tagaattatt ttatctggta gtggtagtat      60

cacggcctat tcccaacaaa cgcggg                                           86


<210>  44
<211>  28
<212>  DNA
<213>  Artificial

<220>
<223>  Reverse Primer

<400>  44
atagatcttt taagtgacga cctccagg                                         28

<210>  45
<211>  23
<212>  DNA
<213>  Artificial

<220>
<223>  Forward Primer

<400>  45
ggcatcttcc gggttgctgt gtg                                              23


<210>  46
<211>  23
<212>  DNA
<213>  Artificial

<220>
<223>  Reverse Primer

<400>  46
cacacagcaa cccggaagat gcc                                              23


<210>  47
<211>  22
<212>  DNA
<213>  Artificial

<220>
<223>  Forward Primer

<400>  47
tccttaaggg agatgtgagc at                                               22


<210>  48
<211>  21
<212>  DNA
<213>  Artificial

<220>
<223>  Reverse Primer

<400>  48
caaggcgatt aagttggtaa c                                                21


<210>  49
<211>  22
<212>  DNA
<213>  Artificial

<220>
<223>  Forward Primer

<400>  49
tccttaaggg agatgtgagc at                                               22


<210>  50

<211>  21
<212>  DNA
<213>  Artificial

<220>
<223>  Reverse Primer

<400>  50
caaggcgatt aagttggtaa c                                                 21


<210>  51
<211>  592
<212>  PRT
<213>  Hepatitis C Virus


<220>
<223>  genotype 1b polymerase NS5B

<400>  51

Met Ser Met Ser Tyr Thr Trp Thr Gly Ala Leu Ile Thr Pro Cys Ala
1               5                   10                  15

Ala Glu Glu Ser Lys Leu Pro Ile Asn Pro Leu Ser Asn Ser Leu Leu
            20                  25                  30

Arg His His Ser Met Val Tyr Ser Thr Thr Ser Arg Ser Ala Ser Leu
        35                  40                  45

Arg Gln Lys Lys Val Thr Phe Asp Arg Met Gln Val Leu Asp Asp His
    50                  55                  60

Tyr Arg Asp Val Leu Lys Glu Met Lys Ala Lys Ala Ser Thr Val Lys
65                  70                  75                  80

Ala Arg Leu Leu Ser Ile Glu Glu Ala Cys Lys Leu Thr Pro Pro His
                85                  90                  95

Ser Ala Lys Ser Lys Phe Gly Tyr Gly Ala Lys Asp Val Arg Ser Leu
            100                 105                 110

Ser Ser Arg Ala Val Asn His Ile Arg Ser Val Trp Glu Asp Leu Leu
        115                 120                 125

Glu Asp Thr Glu Thr Pro Ile Asp Thr Thr Ile Met Ala Lys Asn Glu
    130                 135                 140

Val Phe Cys Val Gln Pro Glu Lys Gly Gly Arg Lys Pro Ala Arg Leu
145                 150                 155                 160

Ile Val Phe Pro Asp Leu Gly Val Arg Val Cys Glu Lys Met Ala Leu
                165                 170                 175

Tyr Asp Val Val Ser Thr Leu Pro Gln Ala Val Met Gly Pro Ser Tyr
            180                 185                 190

Gly Phe Gln Tyr Ser Pro Gly Gln Arg Val Glu Phe Leu Val Asn Thr
        195                 200                 205

Trp Lys Ser Lys Lys Cys Pro Met Gly Phe Ser Tyr Asp Thr Arg Cys
    210                 215                 220

Phe Asp Ser Thr Val Thr Glu Asn Asp Ile Arg Thr Glu Glu Ser Ile
225                 230                 235                 240

Tyr Gln Cys Cys Asp Leu Ala Pro Glu Ala Arg Gln Ala Ile Lys Ser
                245                 250                 255

Leu Thr Glu Arg Leu Tyr Ile Gly Gly Pro Leu Thr Asn Ser Lys Gly
            260                 265                 270

Gln Asn Cys Gly Tyr Arg Arg Cys Arg Ala Ser Gly Val Leu Thr Thr
        275                 280                 285

Ser Cys Gly Asn Thr Leu Thr Cys Tyr Leu Lys Ala Thr Ala Ala Cys
    290                 295                 300

Arg Ala Ala Lys Leu Gln Asp Cys Thr Met Leu Val Asn Gly Asp Asp
305                 310                 315                 320

Leu Val Val Ile Cys Glu Ser Ala Gly Thr Gln Glu Asp Ala Ala Ser
                325                 330                 335

Leu Arg Val Phe Thr Glu Ala Met Thr Arg Tyr Ser Ala Pro Pro Gly
            340                 345                 350

Asp Pro Pro Gln Pro Glu Tyr Asp Leu Glu Leu Ile Thr Ser Cys Ser
        355                 360                 365

Ser Asn Val Ser Val Ala His Asp Ala Ser Gly Lys Arg Val Tyr Tyr
    370                 375                 380

Leu Thr Arg Asp Pro Thr Thr Pro Leu Ala Arg Ala Ala Trp Glu Thr
385                 390                 395                 400

Val Arg His Thr Pro Val Asn Ser Trp Leu Gly Asn Ile Ile Met Tyr
                405                 410                 415

Ala Pro Thr Leu Trp Ala Arg Met Ile Leu Met Thr His Phe Phe Ser
            420                 425                 430

Ile Leu Leu Ala Gln Glu Gln Leu Glu Lys Ala Leu Asp Cys Gln Ile
        435                 440                 445

Tyr Gly Ala Cys Tyr Ser Ile Glu Pro Leu Asp Leu Pro Gln Ile Ile
    450                 455                 460

Glu Arg Leu His Gly Leu Ser Ala Phe Ser Leu His Ser Tyr Ser Pro
465                 470                 475                 480

Gly Glu Ile Asn Arg Val Ala Ser Cys Leu Arg Lys Leu Gly Val Pro
                485                 490                 495

Pro Leu Arg Val Trp Arg His Arg Ala Arg Ser Val Arg Ala Lys Leu
            500                 505                 510

Leu Ser Gln Gly Gly Arg Ala Ala Thr Cys Gly Lys Tyr Leu Phe Asn
        515                 520                 525

Trp Ala Val Arg Thr Lys Leu Lys Leu Thr Pro Ile Pro Ala Ala Ser

530                 535                 540

Gln Leu Asp Leu Ser Gly Trp Phe Val Ala Gly Tyr Asn Gly Gly Asp
545                 550                 555                 560

Ile Tyr His Ser Leu Ser Arg Ala Arg Pro Arg Trp Phe Met Leu Cys
                565                 570                 575

Leu Leu Leu Leu Ser Val Gly Val Gly Ile Tyr Leu Leu Pro Asn Arg
            580                 585                 590


<210>  52
<211>  629
<212>  PRT
<213>  Hepatitis C Virus


<220>
<223>  genotype 1b NS3 full length protein

<400>  52

Ile Thr Ala Tyr Ser Gln Gln Thr Arg Gly Leu Leu Gly Cys Ile Ile
1               5                   10                  15

Thr Ser Leu Thr Gly Arg Asp Lys Asn Gln Val Asp Gly Glu Val Gln
            20                  25                  30

Val Leu Ser Thr Ala Thr Gln Ser Phe Leu Ala Thr Cys Val Asn Gly
        35                  40                  45

Val Cys Trp Thr Val Tyr His Gly Ala Gly Ser Lys Thr Leu Ala Gly
    50                  55                  60

Pro Lys Gly Pro Ile Thr Gln Met Tyr Thr Asn Val Asp Gln Asp Leu
65                  70                  75                  80

Val Gly Trp Pro Ala Pro Pro Gly Ala Arg Ser Met Thr Pro Cys Thr
                85                  90                  95

Cys Gly Ser Ser Asp Leu Tyr Leu Val Thr Arg His Ala Asp Val Ile
            100                 105                 110

Pro Val Arg Arg Arg Gly Asp Ser Arg Gly Ser Leu Leu Ser Pro Arg
        115                 120                 125

Pro Val Ser Tyr Leu Lys Gly Ser Ser Gly Gly Pro Leu Leu Cys Pro
    130                 135                 140

Ser Gly His Val Val Gly Ile Phe Arg Ala Ala Val Cys Thr Arg Gly
145                 150                 155                 160

Val Ala Lys Ala Val Asp Phe Ile Pro Val Glu Ser Met Glu Thr Thr
                165                 170                 175

Met Arg Ser Pro Val Phe Thr Asp Asn Ser Ser Pro Pro Ala Val Pro
            180                 185                 190

Gln Thr Phe Gln Val Ala His Leu His Ala Pro Thr Gly Ser Gly Lys
        195                 200                 205

Ser Thr Lys Val Pro Ala Ala Tyr Ala Ala Gln Gly Tyr Lys Val Leu
    210                 215                 220

Val Leu Asn Pro Ser Val Ala Ala Thr Leu Gly Phe Gly Ala Tyr Met
225                 230                 235                 240

Ser Lys Ala His Gly Ile Glu Pro Asn Ile Arg Thr Gly Val Arg Thr
                245                 250                 255

Ile Thr Thr Gly Gly Pro Ile Thr Tyr Ser Thr Tyr Gly Lys Phe Leu
            260                 265                 270

Ala Asp Gly Gly Cys Ser Gly Gly Ala Tyr Asp Ile Ile Ile Cys Asp
        275                 280                 285

Glu Cys His Ser Thr Asp Trp Thr Thr Ile Leu Gly Ile Gly Thr Val
    290                 295                 300

Leu Asp Gln Ala Glu Thr Ala Gly Ala Arg Leu Val Val Leu Ala Thr
305                 310                 315                 320

Ala Thr Pro Pro Gly Ser Ile Thr Val Pro His Pro Asn Ile Glu Glu
                325                 330                 335

Val Ala Leu Ser Asn Thr Gly Glu Ile Pro Phe Tyr Gly Lys Ala Ile
            340                 345                 350

Pro Ile Glu Ala Ile Lys Gly Gly Arg His Leu Ile Phe Cys His Ser
        355                 360                 365

Lys Lys Lys Cys Asp Glu Leu Ala Ala Lys Leu Thr Gly Leu Gly Leu
    370                 375                 380

Asn Ala Val Ala Tyr Tyr Arg Gly Leu Asp Val Ser Val Ile Pro Thr
385                 390                 395                 400

Ser Gly Asp Val Val Val Val Ala Thr Asp Ala Leu Met Thr Gly Phe
                405                 410                 415

Thr Gly Asp Phe Asp Ser Val Ile Asp Cys Asn Thr Cys Val Thr Gln
            420                 425                 430

Thr Val Asp Phe Ser Leu Asp Pro Thr Phe Thr Ile Glu Thr Thr Thr
        435                 440                 445

Val Pro Gln Asp Ala Val Ser Arg Ser Gln Arg Arg Gly Arg Thr Gly
    450                 455                 460

Arg Gly Arg Ser Gly Ile Tyr Arg Phe Val Thr Pro Gly Glu Arg Pro
465                 470                 475                 480

Ser Gly Met Phe Asp Ser Ser Val Leu Cys Glu Cys Tyr Asp Ala Gly
                485                 490                 495

Cys Ala Trp Tyr Glu Leu Thr Pro Ala Glu Thr Thr Val Arg Leu Arg
            500                 505                 510

Ala Tyr Leu Asn Thr Pro Gly Leu Pro Val Cys Gln Asp His Leu Glu
        515                 520                 525

Phe Trp Glu Ser Val Phe Thr Gly Leu Thr His Ile Asp Ala His Phe
    530                 535                 540

Leu Ser Gln Thr Lys Gln Ala Gly Asp Asn Phe Pro Tyr Leu Val Ala
545                 550                 555                 560

Tyr Gln Ala Thr Val Cys Ala Arg Ala Gln Ala Pro Pro Pro Ser Trp
                565                 570                 575

Asp Gln Met Trp Lys Cys Leu Ile Arg Leu Lys Pro Thr Leu His Gly
            580                 585                 590

Pro Thr Pro Leu Leu Tyr Arg Leu Gly Ala Val Gln Asn Glu Ile Thr
        595                 600                 605

Leu Thr His Pro Ile Thr Lys Phe Val Met Ala Cys Met Ser Ala Asp
    610                 615                 620

Leu Glu Val Val Thr
625


<210>  53
<211>  1176
<212>  DNA
<213>  Respiratory syncytial virus


<220>
<223>  Nucleic acid encoding the Protein N (A.N. D00736, REGION:
        1085..2260)

<400>  53
atggctctta gcaaagtcaa gttaaatgat acattaaata aggatcagct gctgtcatcc      60

agcaaataca ctattcaacg tagtacagga gataatattg acactcccaa ttatgatgtg     120

caaaaacacc taaacaaact atgtggtatg ctattaatca ctgaagatgc aaatcataaa     180

ttcacaggat taataggtat gttatatgct atgtccaggt taggaaggga agacactata     240

aagatactta aagatgctgg atatcatgtt aaagctaatg gagtagatat aacaacatat     300

cgtcaagata taaatggaaa ggaaatgaaa ttcgaagtat taacattatc aagcttgaca     360

tcagaaatac aagtcaatat tgagatagaa tctagaaagt cctacaaaaa actgctaaaa     420

gagatgggag aagtggctcc agaatatagg catgattctc cagactgtgg gatgataata     480

ctgtgtatag ctgcacttgt aataaccaag ttagcagcag gagatagatc aggtcttaca     540

gcagtaatta ggagggcaaa caatgtctta aaaaacgaaa taaaacgcta caagggcctc     600

ataccaaagg atatagctaa cagtttttat gaagtgtttg aaaaacaccc tcatcttata     660

gatgtttttg tgcactttgg cattgcacaa tcatccacaa gagggggtag tagagttgaa     720

ggaatctttg caggattatt tatgaatgcc tatggttcag ggcaagtaat gctaagatgg     780

ggagttctag ccaaatctgt aaaaaatatc atgctaggac atgctagtgt ccaggcagaa     840

atggaacaag ttgtggaagt ttatgagtat gcacagaagt tgggaggaga agctggattc     900

taccatatat tgaacaatcc aaaagcatca ttgctgtcat taactcaatt tcctaacttc     960

tcaagtgtgg tcctaggcaa tgcagcaggt ctaggcataa tgggagagta tagaggtaca    1020

ccaagaaacc aagatctata tgatgcggcc aaagcatatg cagagcaact caaagaaaat    1080

ggagtaataa actacagtgt attagactta acagcagaag aattggaagc cataaagcat    1140

caactcaacc ccaaagaaga tgatgtagag ctttaa                              1176

<210>  54
<211>  391
<212>  PRT
<213>  Respiratory syncytial virus

<220>
<223>  Protein N (A.N. D00736, REGION: 1085..2260)

<400>  54

Met Ala Leu Ser Lys Val Lys Leu Asn Asp Thr Leu Asn Lys Asp Gln
1               5                   10                  15

Leu Leu Ser Ser Ser Lys Tyr Thr Ile Gln Arg Ser Thr Gly Asp Asn
            20                  25                  30

Ile Asp Thr Pro Asn Tyr Asp Val Gln Lys His Leu Asn Lys Leu Cys
        35                  40                  45

Gly Met Leu Leu Ile Thr Glu Asp Ala Asn His Lys Phe Thr Gly Leu
    50                  55                  60

Ile Gly Met Leu Tyr Ala Met Ser Arg Leu Gly Arg Glu Asp Thr Ile
65                  70                  75                  80

Lys Ile Leu Lys Asp Ala Gly Tyr His Val Lys Ala Asn Gly Val Asp
                85                  90                  95

Ile Thr Thr Tyr Arg Gln Asp Ile Asn Gly Lys Glu Met Lys Phe Glu
            100                 105                 110

Val Leu Thr Leu Ser Ser Leu Thr Ser Glu Ile Gln Val Asn Ile Glu
        115                 120                 125

Ile Glu Ser Arg Lys Ser Tyr Lys Lys Leu Leu Lys Glu Met Gly Glu
    130                 135                 140

Val Ala Pro Glu Tyr Arg His Asp Ser Pro Asp Cys Gly Met Ile Ile
145                 150                 155                 160

Leu Cys Ile Ala Ala Leu Val Ile Thr Lys Leu Ala Ala Gly Asp Arg
                165                 170                 175

Ser Gly Leu Thr Ala Val Ile Arg Arg Ala Asn Asn Val Leu Lys Asn
            180                 185                 190

Glu Ile Lys Arg Tyr Lys Gly Leu Ile Pro Lys Asp Ile Ala Asn Ser

195                 200                 205

Phe Tyr Glu Val Phe Glu Lys His Pro His Leu Ile Asp Val Phe Val
    210                 215                 220

His Phe Gly Ile Ala Gln Ser Ser Thr Arg Gly Gly Ser Arg Val Glu
225                 230                 235                 240

Gly Ile Phe Ala Gly Leu Phe Met Asn Ala Tyr Gly Ser Gly Gln Val
                245                 250                 255

Met Leu Arg Trp Gly Val Leu Ala Lys Ser Val Lys Asn Ile Met Leu
            260                 265                 270

Gly His Ala Ser Val Gln Ala Glu Met Glu Gln Val Val Glu Val Tyr
        275                 280                 285

Glu Tyr Ala Gln Lys Leu Gly Gly Glu Ala Gly Phe Tyr His Ile Leu
    290                 295                 300

Asn Asn Pro Lys Ala Ser Leu Leu Ser Leu Thr Gln Phe Pro Asn Phe
305                 310                 315                 320

Ser Ser Val Val Leu Gly Asn Ala Ala Gly Leu Gly Ile Met Gly Glu
                325                 330                 335

Tyr Arg Gly Thr Pro Arg Asn Gln Asp Leu Tyr Asp Ala Ala Lys Ala
            340                 345                 350

Tyr Ala Glu Gln Leu Lys Glu Asn Gly Val Ile Asn Tyr Ser Val Leu
        355                 360                 365

Asp Leu Thr Ala Glu Glu Leu Glu Ala Ile Lys His Gln Leu Asn Pro
    370                 375                 380

Lys Glu Asp Asp Val Glu Leu
385                 390


<210>  55
<211>  1350
<212>  DNA
<213>  Influenza virus A


<220>
<223>  Nucleic acid encoding for Neuraminidase (A.N. AAT72506; VERSION
       AAT72506.1 ; GI:50261907; Accession AY646425.1)

<400>  55
atgaatccaa atcagaagat aataaccatc ggatcaatct gtatggtaat tggaatagtt      60

agcttgatgt tacaaattgg gaacataatc tcaatatggg ccagtcattc aattcagaca     120

gggaatcaac accaagctga accaatcagc aataccaatt ttcttgctga gaaagctgtg     180

gcttcagtaa cattagcggg caattcatct ctttgcccca ttagcggatg ggctgtacac     240

agtaaggaca acggtataag gatcggttcc aagggggatg tgtttgttat aagagagccg     300

ttcatctcat gctcccactt ggaatgcaga actttctttt tgactcaggg agccttgctg     360

aatgacaagc actccaatgg gaccgtcaaa gacagaagcc ctcacagagc attgatgagt 420

tgtcctgtgg gtgaggctcc ctccccatat aactcaaggt ttgagtctgt tgcttggtcg 480

gcaagtgctt gccatgatgg caccagttgg ttgacaattg gaatttctgg cccagacaat 540

ggggctgtgg ctgtattgaa atacaacggc ataataacag acactatcaa gagttggagg 600

aacaacatac tgagaactca agagtctgaa tgtgcatgtg taaatggctc ttgctttact 660

gtaatgactg acggaccaag taatgggcag gcctcatata agatcttcaa aatggaaaaa 720

gggaaagtag ttaaatcagt cgaattggat gcccctaatt atcactatga ggagtgctcc 780

tgttatcctg atgctggcga aatcacatgt gtgtgcaggg ataattggca tggctcaaat 840

cggccatggg tatctttcaa tcaaaatttg gagtatcaaa taggatatat atgcagtgga 900

gttttcggag acaatccacg ccccaatgat ggaacaggca gttgtggtcc ggtgtcccct 960

aacggggcat atggggtaaa agggttttca tttaaatacg gcaatggtgt ttggatcggg 1020

agaaccaaaa gcattaattc caggagcggc tttgaaatga tttgggatcc aaatgggtgg 1080

actggaacgg acagtagctt ctcggtgaaa caagatatcg tagcgataac tgattggtca 1140

ggatatagcg ggagttttgt ccagcatcca gaactgacag gattagattg cataagacct 1200

tgtttctggg ttgagctaat cagagggcgg cccaaagaga gcacaatttg gactagtgga 1260

agcagcatat ctttttgtgg tgtaaatagt gacactgtgg gttggtcttg gccagacggt 1320

gctgagttgc cattcaccat tgacaagtag 1350

<210> 56
<211> 449
<212> PRT
<213> Influenza virus A

<220>
<223> Neuraminidase (A.N. AAT72506; VERSION AAT72506.1; GI:50261907;
 Accession AY646425.1)

<400> 56

Met Asn Pro Asn Gln Lys Ile Ile Thr Ile Gly Ser Ile Cys Met Val
1               5                   10                  15

Ile Gly Ile Val Ser Leu Met Leu Gln Ile Gly Asn Ile Ile Ser Ile
            20                  25                  30

Trp Ala Ser His Ser Ile Gln Thr Gly Asn Gln His Gln Ala Glu Pro
        35                  40                  45

Ile Ser Asn Thr Asn Phe Leu Ala Glu Lys Ala Val Ala Ser Val Thr
    50                  55                  60

Leu Ala Gly Asn Ser Ser Leu Cys Pro Ile Ser Gly Trp Ala Val His

65                  70                  75                  80

Ser Lys Asp Asn Gly Ile Arg Ile Gly Ser Lys Gly Asp Val Phe Val
                85                  90                  95

Ile Arg Glu Pro Phe Ile Ser Cys Ser His Leu Glu Cys Arg Thr Phe
            100                 105                 110

Phe Leu Thr Gln Gly Ala Leu Leu Asn Asp Lys His Ser Asn Gly Thr
        115                 120                 125

Val Lys Asp Arg Ser Pro His Arg Ala Leu Met Ser Cys Pro Val Gly
    130                 135                 140

Glu Ala Pro Ser Pro Tyr Asn Ser Arg Phe Glu Ser Val Ala Trp Ser
145                 150                 155                 160

Ala Ser Ala Cys His Asp Gly Thr Ser Trp Leu Thr Ile Gly Ile Ser
                165                 170                 175

Gly Pro Asp Asn Gly Ala Val Ala Val Leu Lys Tyr Asn Gly Ile Ile
            180                 185                 190

Thr Asp Thr Ile Lys Ser Trp Arg Asn Asn Ile Leu Arg Thr Gln Glu
        195                 200                 205

Ser Glu Cys Ala Cys Val Asn Gly Ser Cys Phe Thr Val Met Thr Asp
    210                 215                 220

Gly Pro Ser Asn Gly Gln Ala Ser Tyr Lys Ile Phe Lys Met Glu Lys
225                 230                 235                 240

Gly Lys Val Val Lys Ser Val Glu Leu Asp Ala Pro Asn Tyr His Tyr
                245                 250                 255

Glu Glu Cys Ser Cys Tyr Pro Asp Ala Gly Glu Ile Thr Cys Val Cys
            260                 265                 270

Arg Asp Asn Trp His Gly Ser Asn Arg Pro Trp Val Ser Phe Asn Gln
        275                 280                 285

Asn Leu Glu Tyr Gln Ile Gly Tyr Ile Cys Ser Gly Val Phe Gly Asp
    290                 295                 300

Asn Pro Arg Pro Asn Asp Gly Thr Gly Ser Cys Gly Pro Val Ser Pro
305                 310                 315                 320

Asn Gly Ala Tyr Gly Val Lys Gly Phe Ser Phe Lys Tyr Gly Asn Gly
                325                 330                 335

Val Trp Ile Gly Arg Thr Lys Ser Ile Asn Ser Arg Ser Gly Phe Glu
            340                 345                 350

Met Ile Trp Asp Pro Asn Gly Trp Thr Gly Thr Asp Ser Ser Phe Ser
        355                 360                 365

Val Lys Gln Asp Ile Val Ala Ile Thr Asp Trp Ser Gly Tyr Ser Gly
    370                 375                 380

Ser Phe Val Gln His Pro Glu Leu Thr Gly Leu Asp Cys Ile Arg Pro
385                 390                 395                 400

Cys Phe Trp Val Glu Leu Ile Arg Gly Arg Pro Lys Glu Ser Thr Ile
                405                 410                 415

Trp Thr Ser Gly Ser Ser Ile Ser Phe Cys Gly Val Asn Ser Asp Thr
            420                 425                 430

Val Gly Trp Ser Trp Pro Asp Gly Ala Glu Leu Pro Phe Thr Ile Asp
        435                 440                 445

Lys

<210>  57
<211>  297
<212>  DNA
<213>  Human immunodeficiency virus 1

<220>
<223>  Nucleic acid encoding for HIV-1 Protease (A.N. CAA09614; VERSION
       CAA09614.1; GI:4375887; embl accession AJ011406.1)

<400>  57
cctcasatca ctctttggca acgacccatc gtcacaataa agataggggg gcaactaaaa      60

gaagctctat tagatacagg agcagatgat acagtattag aagacgtgga tttgccagga     120

agatggaaac caaaaatgat agtgggaatt ggaggttttg tcaaagtaag acagtatgaa     180

gaggtaccca tagaaatctg tggacataaa gttataggta cagtattaat aggacctaca     240

cctgccaatg taattggaag aaatctgtta actcagcttg gctgcacttt aaatttt        297

<210>  58
<211>  99
<212>  PRT
<213>  Human immunodeficiency virus 1

<220>
<221>  misc_feature
<222>  (2)..(2)
<223>  Xaa = non determined

<220>
<223>  HIV-1 Protease (A.N. CAA09614; VERSION CAA09614.1; GI:4375887;
       embl accession AJ011406.1)

<400>  58

Pro Xaa Ile Thr Leu Trp Gln Arg Pro Ile Val Thr Ile Lys Ile Gly
1               5                   10                  15

Gly Gln Leu Lys Glu Ala Leu Leu Asp Thr Gly Ala Asp Asp Thr Val
            20                  25                  30

Leu Glu Asp Val Asp Leu Pro Gly Arg Trp Lys Pro Lys Met Ile Val
        35                  40                  45

Gly Ile Gly Gly Phe Val Lys Val Arg Gln Tyr Glu Glu Val Pro Ile
    50                  55                  60

Glu Ile Cys Gly His Lys Val Ile Gly Thr Val Leu Ile Gly Pro Thr
65                  70                  75                  80

Pro Ala Asn Val Ile Gly Arg Asn Leu Leu Thr Gln Leu Gly Cys Thr
                85                  90                  95

Leu Asn Phe

<210>  59
<211>  15
<212>  PRT
<213>  Artificial

<220>
<223>  NS5B Peptide sensor N21C272

<400>  59

Asp Gly Cys Ala Arg Cys Val Ala Ser Val Gln Leu Tyr Gly Asp
1               5                   10                  15

<210>  60
<211>  15
<212>  PRT
<213>  Artificial

<220>
<223>  NS3 Protease Peptide sensor V7-62

<400>  60

Trp Arg Pro Tyr Tyr Thr Val Leu Cys Ala Leu Ala Ser Trp His
1               5                   10                  15

<210>  61
<211>  15
<212>  PRT
<213>  Artificial

<220>
<223>  NS3 full length peptide sensor H5-34

<400>  61

Pro Ser Asn His Arg Gln Ser Thr Arg Ser Thr Pro Trp Leu Trp
1               5                   10                  15

## Description

The present invention relates to the field of virology. More precisely, the invention provides a method of determining the ability of a test compound to modulate the biological activity of a variant of a target protein, wherein said test compound is previously known to modulate the biological activity of said protein. This invention is useful to determine whether a drug candidate, such as anti-virals, active against a target protein is active against a variant of said protein.

Hepatitis C is a global health problem with 170 million carriers worldwide and 3 to 4 million new cases each year. The virus responsible for this post transfusion non A non B Hepatitis was identified in 1989 as a single stranded RNA virus belonging to the Flaviviridae (Choo et al., 1989). Potential natural histories of HCV include progression from acute infection to long term infection (for 75 to 85% of patients), from long term disease to cirrhosis (for 17 to 20%) and from cirrhosis to decompensation or hepato-cellular carcinoma (for 1 to 5%) (Mc Hutchison, 2004; Walker, 2002). Currently, the standard care consists in a combination between Interferon, a cytokine with immuno-modulatory and antiviral activity (Moussalli et al., 1998) and

Ribavirin, a synthetic guanosine nucleoside analogue (Hugle et al., 2003). For patients infected with HCV genotype 1a/1b (the predominant one in USA, Japan and Europe), the sustained viral response (loss of serum HCV RNA following 24 weeks of antiviral therapy) is at best 42-46% (Walker et al., 2002, Gordon et al., 2005; Lake-Bakaar et al., 2003). Besides its relative inefficacy, this combination therapy yields significant side effects (Fried Michael, 2002). New treatment regimens are needed and investigators have focused on the identification of agents that inhibit specific steps in the virus' lifecycle.

Upon interaction and fusion of viral and cellular membranes, RNA genome is released into the cytoplasm of a newly infected cell and serves as template for RNA replication. Translation of HCV genome depends on an internal ribosome entry site and produces a large polyprotein which is proteolytically cleaved to produce 10 viral proteins. The amino terminal one third of the polyprotein encodes the structural proteins: core protein glycoproteins E1 +E2. After the structural region comes a small integral protein P7 which seems to function as an ion chemical. The remainder of the genome encodes the non structural proteins NS2, N3, NS4A, NS4B, NS5A & NS5B which coordinate the intracellular processes of the virus life cycle (Lindenbach et al., 2005). The development of anti-virals that block essential viral enzymes represents a straight forward approach to developing new anti HCV agents. Although all HCV enzymes are, in theory, equally appropriate for therapeutic intervention, the NS5B RNA polymerase and

NS3-4A serine protease are respectively important for genome replication and polyprotein processing and were the most studied. A variety of biochemical *in vitro* assays have been developed and used in screening campaigns (Behrens et al., 1996; Luo et al., 2000; Oh et al., 1999; Lohmann et al., 1997; Yamashita et al., 1998; Mc Kercher et al., 2004). Although useful, these assays could not predict the enzyme's activity and interaction with inhibitors in a physiological intracellular context. Besides, they don't provide information about the molecule's bioavailability and toxicity.

Until 1999, all cell-based screening efforts for HCV drug discovery relied on surrogate viral systems such as bovine viral diarrhea virus. In 1999, a significant breakthrough in studying HCV RNA replication occurred when the laboratory of R. Bartenschlager developed the HCV replicon system (Lohmann et al., 1999). It became possible to test the effect of inhibitors of traditional targets such as NS3 protease, helicase and NS5B polymerase in an in vitro HCV RNA replication system consisting in a dicistronic, selectable subgenomic HCV replicon (Bartenschlager et al., 2002). The first generation of selectable and reporter-selectable replicons have not been conducive for carrying out high throughput screening due to labor-intensive quantitative RT-PCR methods used in screening and low signal to noise ratio, respectively. In addition, this system requires cell cultures adaptative mutations and still needs improvements of signal window (Blight et al., 2000; Krieger et al., 2001). It also has to face HTS compatibility and rapidness of screening challenges (Bourne et al., 2005; Zuck et al., 2004; O'Boyle et al., 2005) even if a recent publication reported substantial improvements (Hao et al., 2006).

Since HCV demonstrated a high mutation rate in vivo, it was not surprising that some of the molecules under clinical evaluation already induced enzyme specific mutations that lead to decreased sensitivity to the inhibitors used (Sarrazin et al., 2007). Thus, it appears that the resistance profile's study of anti-HCV drugs is essential for their optimization and long term clinical success. Efforts are being made to obtain recombinant NS5B polymerases from several different viral isolates but face problems of physiological relevance. New replicons from clinically relevant viral isolates are under investigation but are still limited to genotypes 1 and 2 and to some cell clones. The observed mutation's implication in the resistance phenotype often needs further investigation (Trozzi et al., 2003; Nguyen et al., 2003). Knowing these techniques' limitations, a cellular screening platform for anti-HCV activity evaluation and resistance profiling is urgently needed. This is the aim of the instant invention.

The inventors now demonstrated that the cell based assay developed by M. Mehtali (WO 2006/046134) is able to detect conformational changes in the target HCV polymerase and protease upon binding to inhibitors. This cell based assay, named 3D-Screen platform, is based on the identification of short peptides that bind specifically the un-liganded target using the two - hybrid system and allow the screening for molecules that dissociate the interaction between the target and the 3D-Sensor peptide. The inventors also demonstrate that this HTS compatible assay enabled the rapid identification of HCV specific anti- NS5B molecules, but in addition is sensitive enough to perform resistance mutants profiling.

A recent study reported constrained peptides that act as inhibitors of NS5B (Amin et al., 2003) but were not used as tools for drug discovery. Several studies used phage affinity selection to identify peptides sensitive to estrogen receptor's conformation (Paige et al., 1999). Another patent WO 02/004956 exploited several peptides' binding to a liganded form of the Estradiol receptor (Fowlkes et al., 2002) to predict the biological activity of a substance by comparing its peptide "fingerprint" to that of a known reference ligand. A very recent publication reported FRET-hybrid interaction methods to screen for peptide ligands capable of recognizing target receptors (You et al., 2006). There was, though, to inventors'knowledges, no report that used peptides as 3D-sensors to screen for drugs directed against the "sensed" HCV polymerase and protease target proteins and their variants thereof.

The instant invention provides an *in vitro* method of determining the ability of a test compound to modulate the biological activity of a variant of a target protein, wherein a ligand is previously known to modulate the biological activity of said target protein, said method comprising the steps of:
Step A) Selecting at least one binding peptide which binds to said target protein and to said variant of the target protein, said method comprising the steps of:
   A1) providing a combinatorial library of peptides where said binding peptide is a member of said library, wherein said library is expressed in a plurality of cells and said cells collectively expressed all members of said library;
   A2) screening said library for the ability of its members to bind to said target protein and to said variant of the target protein, and selecting the peptide(s) binding to said target protein and to said variant of the target protein;
Step B) selecting among the selected peptide(s) of step A2), at least one binding peptide having a decreased or no ability to bind to said target protein in presence of said known ligand and a conserved ability to bind to said variant of target protein in presence of said known ligand.
Step C) testing and selecting a test compound for its ability to decrease the binding of the peptide(s) selected in step B) to said target protein, wherein a decrease or an absence of binding ability is indicative that the test compound induces a conformational change of the target protein, indicating that said test compound modulates the biological activity of said target protein; and
Step D) testing the test compound selected in C) for its ability to modulate the binding ability of the peptide(s) selected in step B) to said variant of the target protein wherein:
   - a decrease or an absence of binding is indicative that the test compound induces a conformational change to said variant of target protein, indicating that said test compound modulates the biological activity of said target protein, said variant of the target protein being not resistant to the modulation of its biological activity by said test compound;
   - a conserved binding is indicative that the test compound does not induce a conformational change to said variant of target protein, indicating that said test compound does not modulate the biological activity of said target protein, said variant of target protein being resistant to the modulation of its biological activity by said test compound.

Preferably the test compounds are small organic molecules, e. g., molecules with a molecular weight of less than 2000 daltons, preferably less than 1000 daltons, more preferably less than 500 daltons, which are pharmaceutically acceptable. Test compounds are preferably of chemical classes amenable to synthesis as a combinatorial library. The test compounds that are selected by the method of the invention includes substances which are similar or "ligand-like" to the substances already identified as having the ability to specifically bind the target protein such as potential pharmacological agonists, antagonists, co-activators and co-inhibitors for the protein in question. Such "ligand-like" test compounds may have a close mechanism of action and a close biological activity to the known-protein ligand, with the potential side effects of the known-protein ligands (agonist or antagonist). More importantly, the test compounds that are selected by the method of the invention includes substances which are not similar to the substances already identified as having the ability to specifically bind the target protein, because the method of the invention identified binding peptides in their native conformation in opposition to the methods of the prior art. Said latter compounds, which are not ligand-like molecule, are expected to have different biological and pharmacological properties compared to the known protein ligand. Alternatively the test compounds may be the binding peptides or a variant thereof, or chemical molecules that mimic said binding peptides, identified by the method of the invention. The test compound of the invention may have been previously identified in a library of chemical molecules by different in vitro assays known by the man skilled in the art, such as for example the cell based assay described in the patent document WO 2006/046134 published on 4 may 2006, particularly in examples4 and 5. The present invention also relates to the test compounds selected by the method of the invention.

The target protein of the invention may be a naturally occurring substance, or a subunit or domain thereof, from any natural source, including a virus, a micro-organism (including bacterial, fungi, algae, and protozoa), an invertebrate (including insects and worms), the normal or pathological cells of an animal, especially a vertebrate (especially a mammal, bird or fish and, among mammals, particularly humans, apes, monkeys, cows, pigs, goats, llamas, sheep, rats, mice, rabbits, guinea pigs, cats and dogs), or the normal or pathological cells of a plant. The target proteins may be alternatively a non-naturally occurring protein that have been in vitro created or modified such as a mutated protein, a chimeric protein or a artificial protein. The target protein may be a glyco-, lipo-, phospho-, or metalloprotein. It may be a nuclear, cytoplasmic, membrane, or secreted protein. It is also an object of the present invention that the target protein, instead of being a protein, may be a macromolecular nucleic acid, lipid or carbohydrate. If it is a nucleic acid, it may be a ribo-or a deoxyribonucleic acid, and it may be single or double stranded. The target protein does not need to be a single macromolecule. The target protein may be a homo or hetero-multimer (dimer, trimer, tetramer, ...) of macromolecules. Additionally, the target protein may necessitate binding partners, such as proteins, oligo-or polypeptides, nucleic acids, carbohydrates, lipids, or small organic or inorganic molecules or ions. Examples include cofactors, ribosomes, polysomes, and chromatin.

The biological activity of the target protein is not limited to a specific activity such as a receptor or an enzymatic activity. Non limiting examples of target proteins include nuclear receptors, orphan nuclear receptor, tyrosine kinase receptors, G-protein coupled receptors, endothelin, erythropoietin receptor, FAS ligand receptor, protein kinases (protein kinase C, tyrosine kinase, serine kinase, threonine kinase, nucleotide kinase, polynucleotide kinase), protein phosphatases (serine/threonine phosphatase, tyrosine phosphatase, nucleotide phosphatase, acid phosphatase, alkaline phosphatase, pyrophosphatase), cell cycle regulators (cyclin cdk2, CDC2, CDC25, P53, RB), GTPases, Rac, Rho, Rab, Ras, endoprotease, exoprotease, metalloprotease, serine protease, cysteine protease, nuclease, polymerase, reverse transcriptase, integrase, ion channels, chaperonins (i.e. heat shock proteins), deaminases, nucleases (i.e. deoxyribonuclease, ribonuclease, endonuclease, exonuclease), telomerase, primase, helicase, dehydrogenase, transferases (peptidyl transferase, transaminase, glycosyltransferase, ribosyltransferase, acetyl transferase, guanylyltransferase, methyltransferase, ...), hydrolases, carboxylases, isomerases, glycosidases, deaminases, lipases, esterases, sulfatases, cellulases, lyases, reductases, ligases, ....

The target proteins of the invention may be structural and non-structural proteins selected among viral proteins, bacterial proteins, vegetal proteins, animal proteins and human proteins. In a preferred embodiment, the target protein is a viral protein. Said viral protein is preferably a hepatitis C virus protein, more preferably a hepatitis C virus protein selected among core protein, glycoproteins E1 and E2, NS1, NS2, NS3, NS4A, NS4B, NS5A, NS5B. Preferably hepatitis C virus proteins are NS5B and NS3. Said viral protein is preferably an influenza virus protein preferably selected among neuraminidase, protein M2 and haemagglutinin. Said viral protein is preferably a lentiviral protein, such as human immunodeficiency virus (HIV) protein, selected among reverse transcriptase, integrase, protease, TAT, NEF, REV, VIF, Vpu, Vpr. Said viral protein is preferably a human respiratory syncytial virus protein preferably selected among proteins N, F and G. Said viral protein is preferably a hepatitis B virus protein preferably the polymerase.

In a second preferred embodiment, said protein is a receptor. The term "receptor" includes both surface and intracellular receptors. Nuclear receptors are of particular interest, specially human nuclear receptor. Nuclear receptors (NR) have been previously described. (NR) are a family of ligand activated transcriptional activators. These receptors are organized into distinct domains for ligand binding, dimerization, transactivation, and DNA binding. The steroid receptor family is a large family composed of receptors for glucocorticoids, mineralo-corticoids, androgens, progestins, and estrogens. Receptor activation occurs upon ligand binding, which induces conformational changes allowing receptor dimerization and binding of co-activating proteins. These co-activators, in turn, facilitate the binding of the receptors to DNA and subsequent transcriptional activation of target genes. In addition to the recruitment of co-activating proteins, the binding of ligand is also believed to place the receptor in a conformation that either displaces or prevents the binding of proteins that serve as co-repressors of receptor function. If the ligand is a pharmacological agonist, the new conformation is one which interacts with other components of a biological signal transduction pathway, e. g.; transcription factors, to elicit a biological response in the target tissue. If the ligand is a pharmacological antagonist, the new conformation is one in which the receptor cannot be activated by one or more agonists which otherwise could activate that receptor. A non exhaustive list of NR is described in WO 2006/046134 (see pages 14 and 15, and figure1). The NR are preferably selected among an estrogen receptor, an androgen receptor, a glucocorticoid receptor, a retinoic acid receptor alpha (RARα), a retinoic X receptor (RXR), a peroxisome proliferators-activated receptor (PPARs), a liver X receptor alpha (LXRα).

According to a preferred embodiment, the target protein and the variant of target protein of the invention are in a native conformation. Preferably, steps A), B), C and D) of the method of the invention are performed with target protein and the variant of target protein in a native conformation. By native conformation, it is meant that the protein is not liganded to its natural ligand. Ligands are substances which bind to target proteins and thereby encompass both agonists and antagonists. However ligands exist which bind target proteins but which neither agonize nor antagonize the receptor. Natural ligands are those ligands, in nature, without human intervention, are responsible for agonizing or antagonizing or binding a natural target protein. The target protein in an un-liganded state has a particular conformation, i. e., a particular 3-D structure. When the protein is complexed to a ligand, the protein's conformation changes.

The term "variant" as used herein encompasses the terms "modified", "mutated", "polymorphisms" "genotypes", quasispecies or "mutant", terms which are interchangeable. The term "variant" refers to a gene or gene product (i.e., target protein) which displays modifications in sequence and or functional properties (i.e., altered characteristics) when compared to the gene or gene product of reference (i.e., target protein); usually, the gene or gene product of reference is the wild-type gene or gene product. The term "wild-type" refers to a gene or gene product which has the characteristics of that gene or gene product when isolated from a naturally occurring source. A wild-type gene is that which is most frequently observed in a population and is thus arbitrarily designed the "normal" or "wild-type" form of the gene. It is noted that naturally-occurring variants can be isolated; these are identified by the fact that they have altered characteristics when compared to the wild-type gene or gene product.

According to the invention, the variant of a target protein includes mutants of said target protein. Non-limiting examples of mutations are point mutations, deletion, insertion, translocation, missense mutations, etc. Usually, the mutation alters the coding sequence of the gene encoding the target protein, and alters the functional properties of said target protein.

According to another embodiment, the variant of the target protein is a natural polymorphism of said target protein. Usually, the polymorphism is one or several modification(s) in the sequence of the gene encoding for said protein, that do not affect significantly the functional properties of said protein (i.e the polymorphism like the mutation is heritable, but not necessarily harmful to the functional properties of the target protein). Polymorphisms in genes and gene products may arise in the general human population. Therefore the present invention, allows determining whether a drug candidate active against a target protein (i.e human nuclear receptors, ...), will be active against a polymorphism of said target protein.

Similarly, the method of the invention allows to determine whether a drug candidate active against a target protein of one virus strain (for example: neuraminidase of influenza virus strain H1N1 or NS5B polymerase of HCV genotype 1), will be active against the homologous protein of another virus strain (i.e neuraminidase of influenza virus strain H3N2 or NS5B polymerase of HCV genotype 2). The present invention will be particularly useful to determine whether a drug candidate against HCV NS5B polymerase or HCV NS3 protease/helicase identified from one HCV genotype, will be also active against, respectively, HCV NS5B polymerase or HCV NS3 protease/helicase from other HCV genotypes.

According to another embodiment, the variant of the target protein is a protein having sequence homology or structural homology with said target protein. Here is a non-limiting example: if the target protein is a protease from HCV, the variant of said target protein may be a human cellular protease having sequence homology or structural homology with said HCV protease. Therefore, the present invention will be particularly useful to determine whether a drug candidate against a viral protease, will be also active against, a human cellular protease; this will be particularly important to evaluate potential toxicity and side effect of a drug candidate. Therefore the method of the invention is useful to predict the toxicity of a drug candidate.

According to a preferred embodiment, the target protein of the invention is the wild-type sequence of hepatitis C virus NS5B protein (genotype 1b) encoded by SEQ ID N° 1 or provided in SEQ ID N° 51. Non-limiting examples of variants of said target protein (i.e., Hepatitis C virus NS5B protein) are M414T, S368A, C316Y, Y448H, P495L, S419M, M423T, S282T, S96T, N142T, G152E, P156L (the second amino acid of SEQ ID N° 51 is considered as the first position for these referenced mutations). According to a second preferred embodiment, the target protein of the invention is the wild-type sequence of hepatitis C virus NS3 protein encoded by SEQ ID N° 2 or provided in SEQ ID N° 52. Non-limiting examples of variants of hepatitis C virus NS3 protein are A156V/S/T, D168A/V, V107A, R155K, T54A, V36M, V36M/A156T, V36A/A156, V36M/R155K, V36T/T54, V36M/T54A, A156V/R155K (the third amino acid of SEQ ID N° 52 is considered as the first position for these referenced mutations). Non-limiting examples of variants of respiratory syncytial virus N protein (see SEQ ID N° 53 and 54) are N105D, I129L, L139I, and I129L with L139I. Non-limiting examples of variants of influenza virus neuraminidase are R292K, D151E, R152K, R371K, R224K, E119G, and D198N (see SEQ ID N° 55 and 56). Non-limiting examples of variants of HIV protease are L10V, I13V, E35G, M36I and H69K (see SEQ ID N° 57 and 58). Non-limiting examples of variants of HIV reverse transcriptase are V179E, T69S, and M184V (see GenBank A.N. AAO84275; GI:37934320; accession AY237815.1 for an example of reference sequence).

The techniques of introducing mutations in proteins are well known to the man skilled in the art and can be done either by biological, physical or chemical means.

A known protein ligand is a substance known to be a ligand for the target protein. The known protein ligand of the invention is able to alter the conformation of the protein upon binding to said protein. Usually, said known protein ligand is a pharmacological agonist or antagonist of a target protein in one or more target tissues of the organism from which the target protein is coming from. However, a known protein ligand may be neither an agonist nor an antagonist of the target proteins. For example, it may be the substrate of the target protein if this latter is an enzyme. The known protein ligand may be, but need not be, a natural ligand of the protein. Said known-protein ligand of the invention is selected among known-target protein agonist and known-target protein antagonist. The known ligand is either endogenously or preferably exogenously added to the cells of step B.

According to a preferred embodiment, the target protein is a hepatitis C viral protein selected among:
a) NS3-4a serine protease, then the known protein ligand is selected among pyrrolidine-5,5-translactam, derivatives of 2,4,6-trihydroxy-3-nitro-benzamides, thiazolidines, benzanilides, BILN2061; ITMN 191, TMC 435350, SCH503034 and VX950;
b) NS3 RNA helicase, then the known protein ligand is selected among 2,3,5-trisubstituted-1,2,4-thiadiazol-2-ium salts, heterocyclic carboxamids and Benzotriazoles;
c) NS5B polymerase, then the known protein ligand is selected among N-1-cyclobutyl-4-hydroxyquinolon-3-yl-benzothiadiazin, 1-{[6-Carboxy-2-(4-chlorophenyl)-3-cyclohexyl-1H-indol-1-yl-]acetyl}-N,N-diethylpiperidin-4-aminium chloride, HCV796, GS9190, MK3281 and VCH 759;
d) other hepatitis C viral proteins. Then, the known protein ligand is selected among ribavirin, levovirin, viramidine, merimpodib, thymosin alpha 1, amantadine.

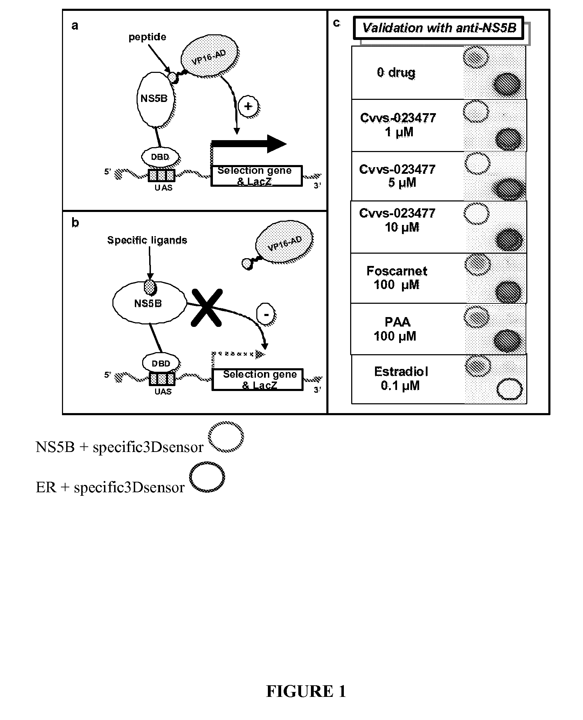

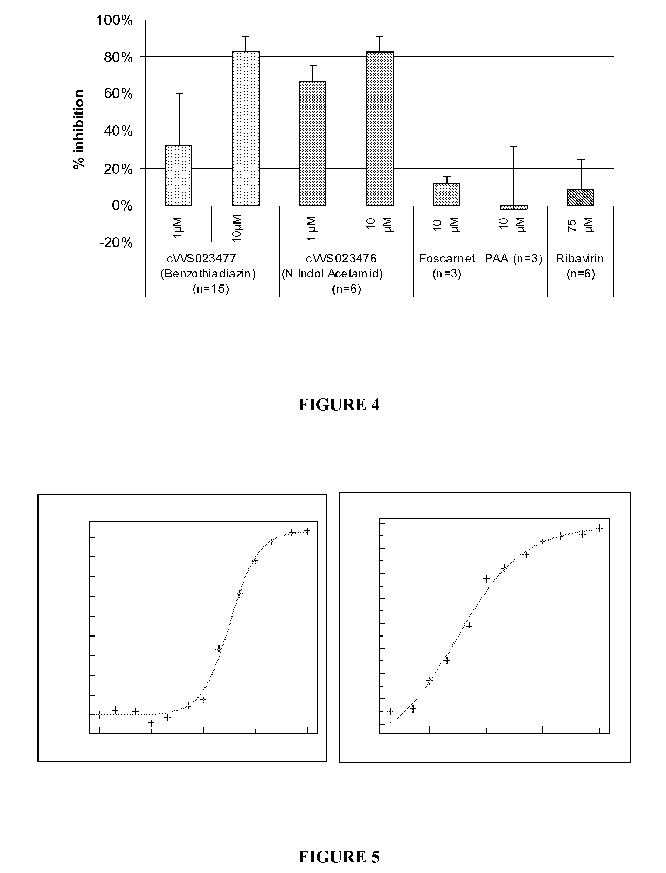

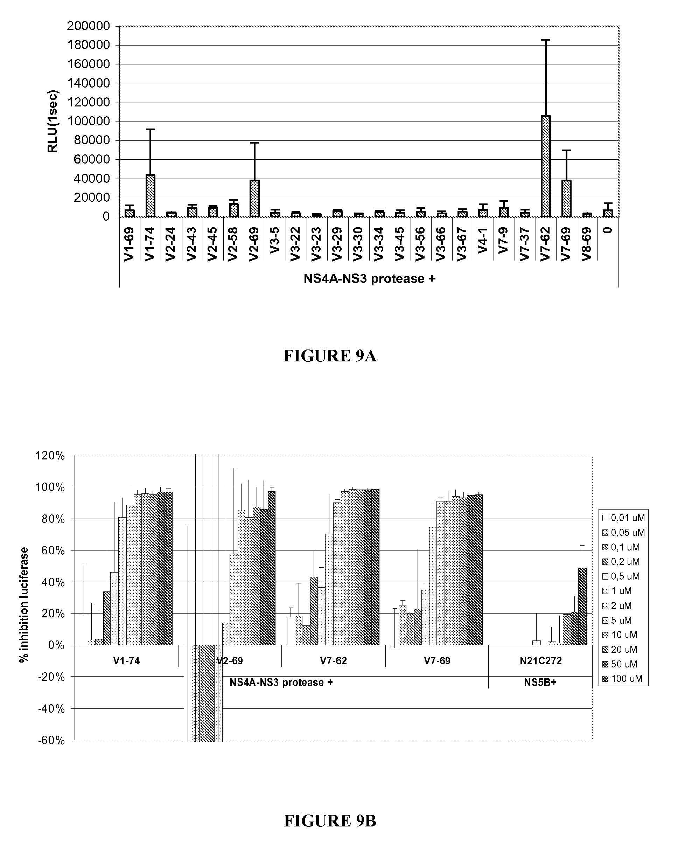

Concerning these above-referenced HCV protein ligands, their structure are well known by the skilled person. For example:
- The BILN 2061 inhibitor corresponds to the compound having the following structure: (1S, 4R, 6S, 7Z, 14S, 18R)-14-cyclopentyloxycarbonylamino-18-[2-(2-isopropylamino-thiazol-4-yl)-7-methoxyquinolin-4-yloxy]-2,15-dioxo-3,16-diazatricyclo[14.3.0.04.6]nonadec-7-ene-4-carboxylic acid (Lamarre et al., 2003);
- The SCH 503034 inhibitor corresponds to the compound having the following structure: (1R,5S)-N-[3-Amino-1-(cyclobutylmethyl)-2,3-dioxopropyl]-3-[2(S)-[[[(1,1-dimethylethyl) amino]carbonyl]amino]-3,3-dimethyl-1-oxobutyl]-6,6-dimethyl-3-azabicyclo[3.1.0]hexan-2(S)-carboxamide (Srikanth Venkatraman et al 2006);
- The VCH-759 inhibitor corresponds to the compound having the structure depicted in the document Beaulieu P.L. et al. 2007, page 621 figure 9);
- The MK0608 inhibitor can be also cited (see Beaulieu et al., 2007, page 616, figure 4);
- The TMC435350 inhibitor corresponds to the compound having the structure depicted in the document Simmen K et al., 2007, figure 1 du poster P-248 poster disclosed in the "14th international symposium on hepatitis C, Glasgow September 2007" (Figure 1);
- The ITMN 191 inhibitor corresponds to the compound having the structure depicted in the document Manns et al., 2007 (Table 1, page 996, Ref. 85); and
- The GS 9190 inhibitor (from Gilead) (see Sheldon J. et al, 2007, Expert Opinion on Investigational Drugs, Vol. 16, No. 8, Pages 1171-1181).

According to another preferred embodiment, the target protein is an influenza virus viral protein selected among:
a) neuraminidase, then the known-protein ligand is selected among zanamivir and oseltamivir, and
b) protein M2, then the known protein ligand is selected among amantadine and rimantadine.

According to another preferred embodiment, the target protein is an HIV viral protein selected among:
a) viral protease, then the known protein ligand is selected among amprenavir, indinavir, saquinavir, lopinavir, fosamprenavir, ritonavir, atazanavir, nelfinavir; and
b) reverse transcriptase, then the known protein is selected among lamivudine, zalcitabine, delavirdine, zidovuline, efavirenz, didanosine, nevirapine, tenofovir disoproxil fumarate, abacavir, stavudine.

According to a preferred embodiment, the target protein is a nuclear receptor selected among:
a) estrogen receptor, then the known-protein ligand may be selected among: estradiol, diethylstilbestrol, genistein, tamoxifen, ICI182780 (Faslodex®), raloxifen; and
b) androgen receptor, then the known-protein ligand may be selected among: testosterone, dihydrotestostreone, progesterone, medroxyprogesterone acetate, cyproterone acetate, mifepristone, dehydroepiandrosterone, flutamide; and
c) glucocorticoid receptor, then the known-protein ligand may be selected among:
   dexamethasone, medroxyprogesterone acetate, cortivazol, deoxycorticosterone, mifepristone, fluticasone propionate, dexamethasone; and
d) Peroxisome proliferators-activated receptors (PPARs), then the known-protein ligand may be selected among the glitazones such as troglitazone; and
e) Liver X Receptor alpha (LXRα), then the known-protein ligand may be T1317 (Tularik®); and
f) Retinoic acid receptor (RAR), then the known-protein ligand may be selected among:
   all-trans retinoic acid, 9-cis-retinoic acid; and
g) Retinoid X receptor (RXR) then the known protein ligand may be selected at among:
   all-trans retinoic acid, 9-cis-retinoic acid.

The main limitation in the method of selecting binding peptides of the invention is the availability of known ligands (i.e drugs) to identify conformation sensitive binding peptides. In the virus field for example, it is often the case that no antiviral drug is known to bind to a viral target protein (i.e respiratory syncythial virus). However, since viruses are intracellular parasites which are using the cellular machinery for their replication, viral proteins are interacting with host cell proteins or molecules (ATP, tRNA, ribosomes, cell enzymes, ...). Interactions of numerous virus proteins with those of the host cells have been reported in the scientific literature (Tellinghuisen & Rice Curr Opin Microbiol., 2002, 5:419-427; HIV-1, Human proteins interaction database, National Institute of Allergy & Infectious Diseases; Lo et al., J Virol., 1996 Aug. 70(8):5177-82). According to another embodiment of the invention, when no ligand (i.e. pharmacological agonists or antagonists) is known to bind to the target proteins, either cellular or viral proteins interacting with the target protein might be used instead to identify conformation sensitive binding peptides. Once peptides interacting with the target protein are identified, an expression vector encoding the protein interacting with the target protein is introduced in the yeast already modified with an expression vector encoding the target protein and an expression vector encoding the peptide library. Peptides that will be blocked by the protein/protein interaction, either due to the conformation modification of the target or due to the inaccessibility of their recognition site on the target protein due to a steric hindrance, will be identified. Therefore, the invention also relates to a method of selecting binding peptide(s) (steps A) and B) of the present invention) which binds a target protein in a native conformation, said method comprising the steps of:
a) providing a combinatorial library of peptides where said binding peptide is a member of said library, wherein said library is expressed in a plurality of cells and said cells collectively expressed all members of said library;
b) screening said library for the ability of its members to bind said target protein and to said variant of the target protein, and selecting the peptide(s) binding to said target protein and to said variant of target protein;
c) screening of peptides selected in b) for the ability to bind to said protein in presence of one protein known to interact with the target protein; and
d) selecting the peptides screened in c) having a decrease ability or no ability to bind to said target protein in presence of said protein known to interact with the target protein, and a conserved ability to bind to said variant of target protein in presence of said protein known to interact with the target protein.
If proteins interacting with the target protein are not known, the man of the art knows how to identify proteins that are able to interact with a target protein. The yeast two-hybrid is a validated technology to identify protein-protein interactions. For example, cDNA clones encoding proteins interacting with the target protein can be isolated with a yeast two-hybrid screen of a cDNA library from human cells of interest. Human cDNA library could be commercially available or will have to be constructed.

By the terms "bind" or "binding", it is meant herein that the peptide and the target protein interact, attach, join or associate to one another sufficiently that the intended assay can be conducted. By the terms "specific" or "specifically", it is meant herein that the peptide and the target protein bind selectively to each other and not generally to other components unintended for binding to the subject components. The parameters required to achieve specific interactions can be determined routinely, e.g. using conventional methods in the art.

The term "library" generally refers to a collection of chemical or biological entities which are related in origin, structure, and/or function, and which can be screened simultaneously for a property of interest. The term "combinatorial library" refers to a library in which the individual members are either systematic or random combinations of a limited set of basic elements, the properties of each member being dependent on the choice and location of the elements incorporated into it. Typically, the members of the library are at least capable of being screened simultaneously. Randomization may be complete or partial; some positions may be randomized and others predetermined, and at random positions, the choices may be limited in a predetermined manner. The members of a combinatorial library may be oligomers or polymers of some kind, in which the variation occurs through the choice of monomeric building block at one or more positions of the oligomer or polymer, and possibly in terms of the connecting linkage, or the length of the oligomer or polymer, too. Or the members may be non-oligomeric molecules with a standard core structure with the variation being introduced by the choice of substituents at particular variable sites on the core structure. Or the members may be non-oligomeric molecules assembled like a jigsaw puzzle, but wherein each piece has both one or more variable moieties (contributing to library diversity) and one or more constant moieties (providing the functionalities for coupling the piece in question to other pieces). In a "simple combinatorial library", all of the members belong to the same class of compounds (e. g., peptides) and can be synthesized simultaneously. A "composite combinatorial library" is a mixture of two or more simple libraries, e. g., DNAs and peptides, or chemical compound. Preferably, a combinatorial library will have a diversity of at least 100, more preferably at least 1,000, still more preferably at least 10,000, even more preferably at least 100,000, most preferably at least 1,000,000, different molecules. The peptide library of the invention is a combinatorial library. The members of this library are peptides having at least three amino acids connected via peptide bonds. Preferably, they are at least four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, or thirty amino acids in length. Preferably, they are composed of less than 50, preferably less than 25 amino acids and more preferably less than 15. The peptides may be linear, branched, or cyclic, and may include non-peptidyl moieties. The amino acids are not limited to the naturally occurring amino acids.

The cells of the invention are cells which, naturally or not, functionally express the suitable target protein. Preferably, the cells are eukaryotic cells. The cells may be from a unicellular organism, a multi-cellular organism or an intermediate form (slime mold). If from a multi-cellular organism, the latter may be an invertebrate, a lower vertebrate (reptile, fish, amphibian), a higher vertebrate (bird, mammal) or a plant. According to a preferred embodiment, the cell of steps A), B), C) and D) of the method of the invention is a cell, preferably isolated cell, not integrated into a whole multi-cellular organism or a tissue or organ of an organism.

According to a preferred embodiment, the cells used to select the binding peptides (steps A) and B) of the method of the invention) are non-mammalian eukaryotic cells, preferably a yeast cell. Preferably, the yeast cells are of one of the following genera: Saccharomyces, Schizosaccharomyces, Candida, Hansenula, Pichia, Kluyveromyces, Cryptococcus, Yarrowia and Zygosaccharomyces. More preferably, they are of the species: Saccharomyces cerevisiae. Other non-mammalian cells of interest include plant cells (e.g. arabidopsis), arthropod cells, annelid or nematode cells (e. g., Caenorhabditis elegans; planaria; leeches; earthworms; polychaetus annelids), crustaceans (e. g., daphnia), protozoal cells (e. g. Dictyostelium discoideum), and lower vertebrate (reptiles, amphibians, fish) cells.

According to a preferred embodiment, the cell used to select and test the test compounds (steps C) and D) of the method of the invention) is a mammalian cell of animal or human origin. Non-limiting examples of mammalian cells are human primary cells, human embryonic cells, human cell lines. In a preferred embodiment, human cells are continuous human cell lines; non-limiting examples of human cell lines includes Hela, Huh7, T47D, A549, HEK-293, MCF7. Alternatively, said human cells are embryonic cells, preferably embryonic cells selected among totipotent cells (i.e. Embryonic Stem cells, ES cells), pluripotent, multipotent and unipotent cells. In a specific embodiment, the cell is a human ES cells and the target protein is a key protein involve in the control of a differentiation pathway of ES cells to human differentiated cells. In a second embodiment, said cells are selected among animal cells such as animal primary cells, animal embryonic cells and animal cell lines. Non limiting examples of animal cell lines include VERO, CHO, NSO, COS, MDCK, MDBK and 3T3. When the target protein is an hepatitis C or B viral protein, the cells used to select and test the test compounds (steps C) and D) of the method of the invention) is preferably animal or human hepatocytes, or hepatocyte cell lines. Non limiting examples of hepatocyte cell lines are Huh7, HepG2, and HepaRG. When the target protein is an HIV viral protein, the cells used to select and test the test compounds (steps C) and D) of the method of the invention) is preferably animal or human lymphocytes or lymphoblastoid cell lines, such as HL60, U937, TF-1, K562 and IM-9. When the target protein is an influenza or RSV viral protein, the cells used to select and test the test compounds (steps C) and D) of the method of the invention) is preferably animal or human epithelial cell lines. Non limiting examples of lung cell lines are A549 and HEp-2.

Each cell used to select the binding peptides (steps A) and B) of the method of the invention) cell is co-expressing:
- said target protein or said variant of target proteins, or a ligand-binding protein moiety thereof, and
- one member of said combinatorial library of peptides, and each cell is further providing a signal producing system operably associated with said target protein or variant of target proteins, or moiety, such that a signal is produced which is indicative of whether said member of said library binds said target protein or moiety in or on said cell.

Preferably, said signal generated by the reporter gene expression is a quantitative signal which can be measured.

Preferably, the total quantity of said signal which is measured is proportional to the number of peptide specifically bound to the target protein or to a variant thereof, resulting to the measure of an increased, a conserved, a decrease or an absence of binding (compared to a control or referenced sample).

A signal is produced when said peptide binds to said target protein and to said variant of target protein. Said signal is decreased or absent when said peptide has a decreased ability or is unable to bind to said target protein or to said variant of target protein, liganded to a known ligand to said target protein.

The cell used to select and test the test compounds (steps C) and D) of the method of the invention) is co-expressing:
a) said target protein or said variant of target protein, or a ligand-binding protein moiety thereof; and
b) said peptide selected in step B) and able to bind to the target protein and to said variant of target protein in absence of known ligand; and said cell is further providing a signal producing system operably associated with said target protein or said variant of target protein, or a ligand-binding protein moiety thereof whereby:
   - the binding of said peptide to said protein in presence of test compound results in the constitution of a functional transactivation activator protein which activate expression of said reporter gene, whereby a signal is produced which is indicative that said peptide binds said target protein or said variant of target protein, or moiety, in or on said cell of steps C) and D), and that said test compound does not modify the conformation of said target protein or variant of target protein; or
   - the decrease or the absence of binding of said peptide to said protein in presence of the test compound does not allow the constitution of a functional transactivation activator protein, whereby no signal is produced which is indicative that said test compound modify the conformation of said target protein or variant of said target protein.

The signal producing system of the method of the invention is selected among signal producing system endogenous to the cell and signal producing system exogenous to the cell. Preferably, said signal producing system is endogenous to the cell. The signal producing system comprises a protein-bound component which is fused to said target protein or moiety so as to provide a chimeric protein and a peptide-bound component which is fused to said peptide so as to provide a chimeric peptide, whereby a signal is produced when the peptide-bound and protein-bound components are brought into physical proximity as a result of the binding of the peptide to the target protein. Said components is a DNA-binding domain (DBD) and another of said components is a complementary transactivation domain (AD), and the signal producing system further comprises at least one reporter gene operably linked to an operator bound by said DNA-binding domain, the binding of the peptide to the target protein resulting in the constitution of a functional transactivation activator protein which activates expression of said reporter gene. Said signal producing system of the invention comprises:
(i) a complementary transactivation domain (AD) which is fused to said peptide to provide a chimeric peptide. The AD is preferably selected from the group consisting of *E*. *coli* B42, Gal4 activation domain II, and HSV VP16; and
(ii) a DNA-binding domain (DBD) which is fused to said target protein to provide a chimeric protein; the DBD is preferably selected from the group consisting of Gal4 and LexA; and
(iii) a signal producing system comprising at least one reporter gene operably linked to an operator bound by said DBD,
   whereby the binding of said peptide to said protein, results in the constitution of a functional transactivation activator protein which activate expression of said reporter gene, whereby a signal is produced which is indicative of the binding of said peptide to said target protein, or a variant of target-protein, or a ligand-binding protein moiety thereof, in or on said cell used in steps A), B), C) or D).

A signal is "produced" when said signal is detectable by the means adapted and normally used by the man skilled in the art to perform the detection (i.e. human eye, spectrophotometer, etc.). The indication that an absence of binding had occurred is when the signal is no longer detectable by the means routinely used to detect the reporter gene expression by the man skilled in the art. The indication that a decrease in the binding had occurred is when the decrease of the signal generated by the reporter gene expression is at least 50 %, preferably at least 75 %, and more preferably at least 90 % of the signal generated when the specific binding occurred.

When the signal is the death or survival of the cell in question, the assay is said to be a selection. When the signal merely results in detectable phenotype by which the signalling cell may be differentiated from the same cell in a non-signalling state, the assay is a screen. However the term "screening" in the present invention may be used in a broader sense to include a selection. According to the present invention, "reporter gene" means a gene, the expression of which in one cell, allows to detect said cell in a large population of cells; that is to say, that it allows to distinguish between the cells that are expressing or not said reporter gene. The reporter gene of the invention includes detectable phenotype reporter genes and resistance selection genes. Non-limiting examples of resistance selection genes include yeast genes: 3-isopropylmalate dehydrogenase (LEU2), phosphoribosylanthranilate isomerase (TRP1), imidazole-glycerol-phosphate dehydratase (HIS3), Orotidine-5'-phosphate (OMP) decarboxylase (URA3) and antibiotic resistance genes. Among the antibiotics, one can recite, neomycin, tetracycline, ampicilline, kanamycine, phleomycine, bleomycine, hygromycine, chloramphénicol, carbenicilline, geneticine, puromycine, blasticidine. The antibiotics resistance genes are well known to the man skilled in the art. For example, neomycine gene provides the cells with a resistance to G418 antibiotic added to the cell culture medium. Alternatively, non-limiting examples of detectable phenotype reporter genes include the following genes: DHFR, luciferase, chloramphenicol acetyltransferase, beta-lactamase, adenylate cyclase, alkaline phosphatase, and beta-galactosidase, and auto-fluorescent proteins (such as green fluorescent protein, red fluorescent protein, blue fluorescent protein, yellow fluorescent protein, and all the variants and derived fluorescent proteins). The signal producing system may include, besides reporter gene(s), additional genetic or biochemical elements which cooperate in the production of the signal. Such an element could be, for example, the substrate of a reporter gene which is an enzyme.

There may be more than one signal producing system, and the system may include more than one reporter gene.

According to a preferred embodiment, the method of selecting binding peptides (steps A) and B) of the method of the invention)) is performed in yeast cells, and the signal producing system is comprising at least two reporter genes in tandem, one resistance selection gene selected among HIS3, LEU2, TRP1, URA3 and one reporter gene selected among luciferase, auto-fluorescent proteins and beta-galactosidase. In another preferred embodiment, the method of selecting test compounds of the invention (steps C) and D) of the method of the invention)) is performed in mammalian cells, preferably a human cell, and the signal producing system is comprising at least one reporter gene selected among luciferase, auto-fluorescent proteins and beta-galactosidase. The test compound is endogenously or, preferably, exogenously added to the cell of steps C) and D).

The present invention is useful to predict whether a candidate drug will be active or not against a target protein and the variants of such target protein and to select the drug candidate with the broadest scope of action.

The invention also relates to the test compounds selected by the method of the invention. The selected test compounds of the invention may be used as an assay hit for the identification of hits which constitute new candidate molecule(s) for drug development. The invention also relates to the use of selected test compounds of the invention as a drug with a broad spectrum of action against target protein wild type, target protein polymorphisms, target protein resistance mutants, especially for the prophylactic and therapeutic treatment of diseases, such as, without limitation and as examples, human and animal viral diseases and human and animal cancers.

The invention is also directed to an isolated peptide selected from the group consisting of:
- DGCARCVASVQLYGD (SEQ ID N° 59);
   - WRPYYTVLCALASWH (SEQ ID N° 60); and
   - PSNHRQSTRSTPWLW (SEQ ID N° 61), or recombinant vector or host cell comprising said peptide.

The invention also relates to the method for determining the ability of a test compound to modulate the biological activity of a variant of a hepatitis C virus protein according to the present invention **characterized in that** said methods comprises a step wherein the test compound is tested for its ability to decrease the binding of the peptide:
- DGCARCVASVQLYGD (SEQ ID N° 59) when the target protein is the HCV NS5B;
- WRPYYTVLCALASWH (SEQ ID N° 60) when the target protein is the HCV NS3 protease; and
- PSNHRQSTRSTPWLW (SEQ ID N° 61), when the target protein is the HCV full length NS3.

For the remainder of the description, reference will be made to the legend to the figures below.

### FIGURES

### Figure 1: Identification of conformation sensitive peptides interacting with the NS5BD21

(a) Isolation of peptides interacting with the NS5BD21 in the yeast two-hybrid-system. Yeast cells carrying the bait (NS5BD21-LexA) were transformed with the prey represented by a library of random peptides (in frame with the viral VP16 activation domain). Colonies growing on selective medium and staining blue allowed the isolation of individual peptides that would specifically interact with the polymerase. (b) Isolation of conformation sensitive peptides. The interacting peptides were then tested in presence of ligands known to induce conformational changes in the bait NS5B. The "3D-sensors" were thus identified as the ones whose binding to the target polymerase was prevented by known anti-NS5B. The corresponding colonies didn't show any expression of the selection or reporter gene. (c) Characterization of NS5BD21 "3D-sensors" in a spot test. Here were represented 2 selected yeast colonies: one carrying the ER and its specific peptide called N° 30-2 isolated previously and used as a control, the other carrying the NS5BD21 and its specific peptide called N21C272. Both clones were spotted on selective medium lacking histidine, leucine and tryptophane, supplemented or not with polymerase ligands and overlayed with an X-Gal staining. The NS5BD21+N21C272 clone grew on selective medium in the absence of any NS5B ligand and stained positive for LacZ. Colonies issued from the same culture didn't show any growth on selective medium containing varying amounts of specific NS5B inhibitor (Benzothiadiazin CVVS023477). Non specific viral polymerase inhibitors (Foscarnet, PAA) or unrelated ligands (Estradiol) didn't show any effect on growth or LacZ staining. Our control clone (E positif specific 3D sensor N° 30.2) was sensitive to its corresponding specific ligand (Estradiol) but not to NS5B or other viral polymerase inhibitors.

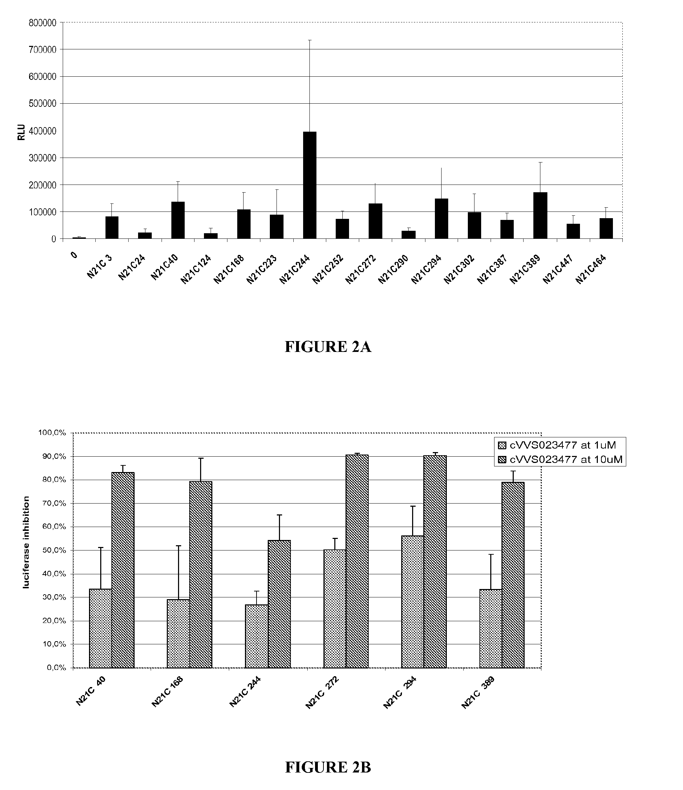

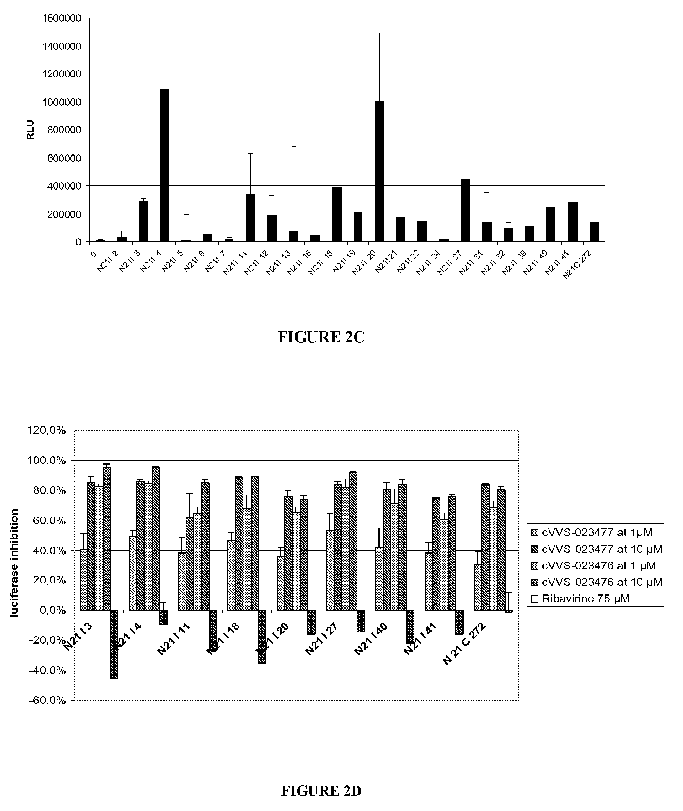

### Figures 2A, 2B, 2C and 2D: Validation of the 3D-sensors in mammalian cells

Hela cells were co-transfected with plasmids expressing: (i) the 3D-sensor isolated in yeast fused to the trans-activation domain (VP16-AD); (ii) the target polymerase (NS5BD21 fused to the yeast Gal4-DBD) and (iii) the luciferase reporter gene whose expression is inducible by the complex [NS5BD21/3D-sensor-VP16].
Figure 2A: Luciferase activation by the 3D-Sensors isolated in yeast with the Benzothiadiazidic drug. (N=4) Measurements of luciferase expression in the absence of any drug were represented for the sixteen 3D-sensors isolated in yeast using the reference drug cVVS-023477. Cells carrying the three plasmids showed a strong activation of the luciferase reporter for 6 of the tested peptides with a ratio signal over background greater than 20.
Figure 2B: Reactivity of the most potent 3D-Sensors to the reference Benzothiadiazidic drug.(N=4) Percentages of luciferase inhibition in the presence of the Benzothiadiazidic NS5B specific ligand cVVS-023477 were represented for the six 3D-sensors presenting the best luciferase activation (ratio signal over background greater than 20). The 3D-sensor N21C272 was selected for its better responsiveness to dissociation by a specific anti-NS5B (51% luciferase inhibition at 1uM and 90% at 10uM final concentration) and the least data variability.
Figure 2C: Luciferase activation by the 3D-Sensor isolated in yeast using the Indol drug (N=3) Measurements of luciferase expression in the absence of any drug were represented for the twenty two 3D-sensors isolated in yeast using the reference drug cVVS-023476. Cells carrying the three plasmids showed a strong activation of the luciferase reporter for 8 of the tested peptides with a ratio signal over background greater than 20.
Figure 2D: Reactivity of the most potent 3D-Sensors to the Indol and Benzothiadiazidic reference drugs (N=3) Percentages of luciferase inhibition in the presence of the Indol Acetamid NS5B specific ligand cVVS-023476 or the Benzothiadiazidic NS5B specific ligand cVVS-023477 were represented for the eight 3D-sensors presenting the best luciferase activation (ratio signal over background greater than 20).

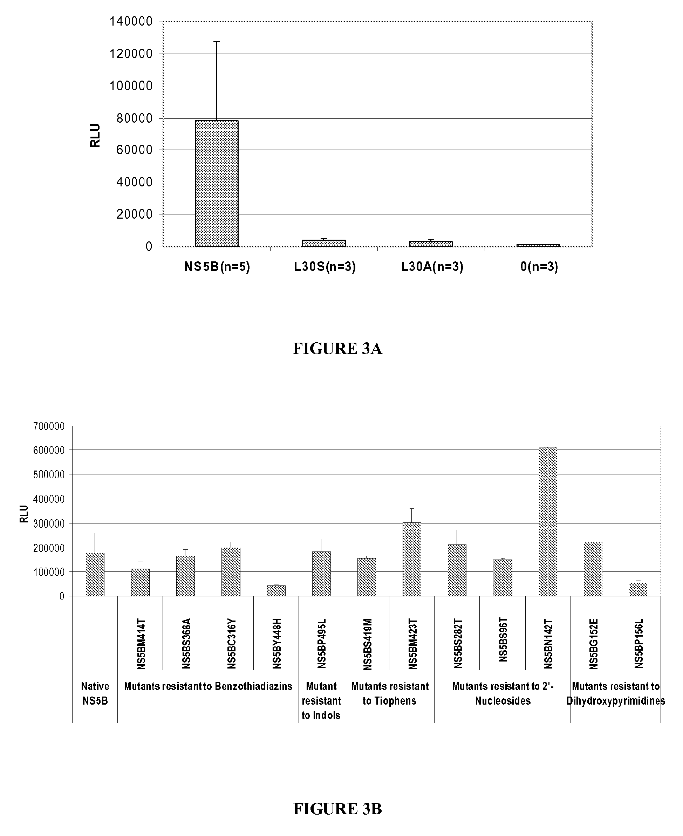

### Figures 3A and 3B: Selectivity of the selected 3D-sensor towards the target

Hela cells were transiently transfected with the luciferase reporter, the 3D- and vectors expressing either native NS5BD21 or different mutants. Measurements of luciferase expression in absence of any ligand were represented.
Figure 3A: Luciferase activation by the 3D-Sensor in presence of native NS5B or structure destabilising mutants. Measurements of luciferase expression in absence of any drug showed that the interaction between with the 3D-sensor N21C272 didn't occur with the L30S and L30R conformational destabilizing mutants described by Labonté P (2002).
Figure 3B: Luciferase activation by the selected 3D-Sensor in presence of a variety of resistance mutants. The 3D-sensor N21C272 did bind to 10 of the 12 drug-resistant mutants tested : the M414T, S286A and C316Ymutants described as resistant to Benzothiadiazins, the Indol resistant mutant P495L, the Tiophens resistant mutants S419M and M423T, the 2' nucleoside analogues resistant mutants S282T, S96T and N142T and the G152E mutant resistant to Dihydroxypyrimidines.

### Figure 4: Drugs' specificity of action on the 3D-Screen platform

Hela cells were transiently transfected with the luciferase reporter, the 3D-sensorN21C272 and the fusion protein NS5BD21-Gal4 then incubated for 24 hours with either specific or non specific ligands. Percentages of inhibition of resulting luciferase's expression were calculated versus an equivalent amount of solvent (DMSO). NS5B specific inhibitor cVVS-023477 (Benzothiadiazin (Pratt JK 2005)) induced a strong inhibition of the luciferase signal at 10 µM final concentration (83.2% +/- 7.5; n=15) while another specific NS5B inhibitor cVVS-023476 (Indol-N-Acetamid (Harper S 2005)) showed a noticeable effect starting at 1 µM final concentration(67% +/- 8.3, n=6). Non specific ligands such as Foscarnet or Phosphonoacetic acid didn't yield any significant signal inhibition at 10 µM concentration (respectively 11.7% +/- 4 and -2.2% +/-33.8; n=3). Another important negative control was Ribavirin, whose presumed mechanism of action seems not to be related to a direct NS5B inhibition (Lohmann V, 2000; Maag D, 2001), didn't show any noticeable effect on our system at 75 µM final concentration (inhibition 8.7% +/- 16.3; n=6).

### Figure 5: Concentration-response relationship for NS5B ligands

Percentages of luciferase's inhibition were represented for cells transfected with plasmids encoding the reporter luciferase, the NS5BD21-Gal4 fusion protein and the 3D-sensor N21C272-VP16 after incubation in presence of increasing concentrations of either cVVS023477 or cVVS023476 (between 0 and 100 µM final concentration) for 24 hours. The obtained curve showed a hyperbolic relationship in agreement with the Michaelis-Menten model.

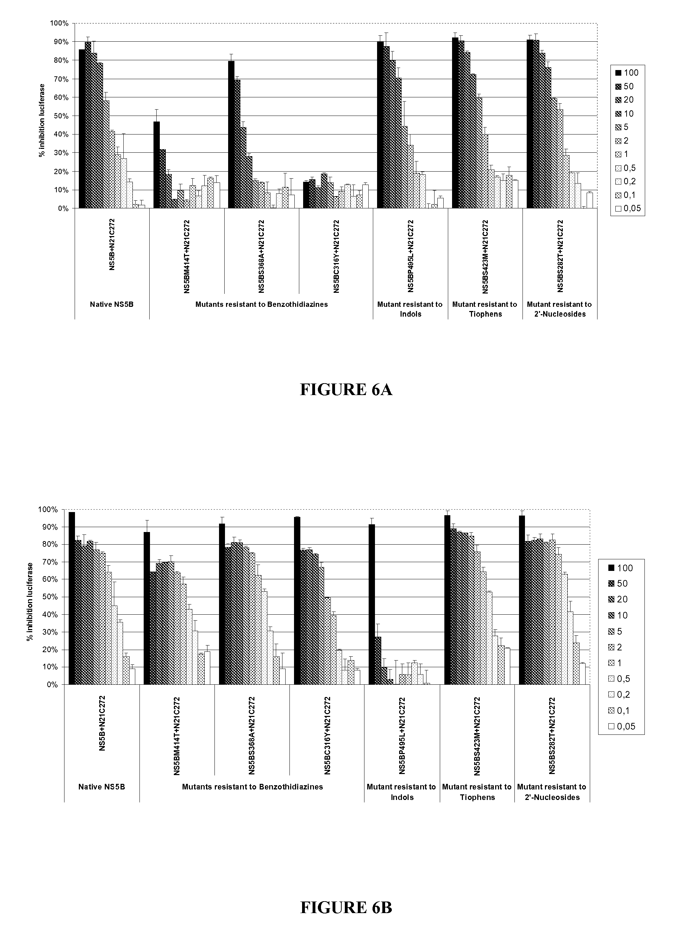

### Figures 6A and 6B: Mutant 3D-Screen platform's reactivity to reference drugs

Hela cells were transiently transfected with the luciferase reporter, the 3D-sensorN21C272 and vectors expressing either native NS5BD21 or different mutants fused in frame with the Gal4 DNA-binding domain. Percentages of reporter's inhibition were represented for cells incubated with a specific anti-NS5B at increasing concentrations for 24 hours.
Figure 6A: Reactivity of different mutants 3D-Screen platform to the Benzothiadiazidic compound. For cells expressing either the native NS5B or the Benzothiadiazidic sensitive mutants P495L, S423M or S282T mutants sensitive to Benzothiadiazins, the benzothiadiazidic compound yielded a comparable luciferase inhibition with an IC50 between 2 and 5 µM (n=2). On the other hand, the same compound didn't show any noticeable reporter inhibition for cells transfected with the Benzothiadiazidic resistant mutants M414T, S368A or G316Y.
Figure 6B: Reactivity of different mutants 3D-Screen to the Indol compound. For cells expressing either the native NS5B or the Indol sensitive mutants, the Indol N Acetamid compound inhibited the generated signal with an IC50 between 0.2 and 2µM while it didn't for the P495L one that was described as resistant to Indols (the IC50 is at least 10 fold higher) (n=2).

One can notice that the 3 mutants resistant to Benzothidiazins challenged on figure 6A react to Indols' action like other Indol sensitive mutants.

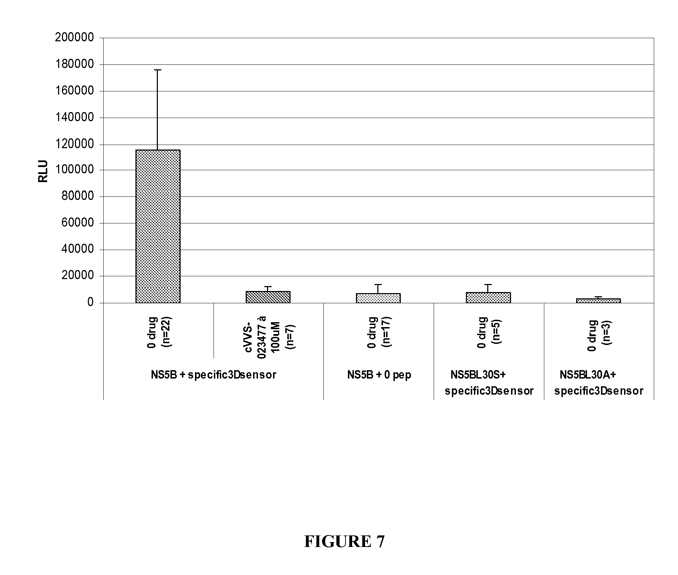

### Figure 7: Assay's signal window: Hela cells were transiently transfected with the luciferase reporter, the 3D-sensorN21C272 and vectors expressing either native NS5BD21 or different mutants

Measurements of luciferase expression, in presence or absence of specific ligands, were represented. The background signal emitted by cells transfected with the native NS5BD21 and a backbone vector without the 3D-sensor-VP16 7182 RLU +/- 6643 (n=17) while an equivalent one was obtained with a denaturated NS5B (L30S and L30A conformational destabilizing mutants described by Labonté et al., 2002) in presence of the 3D-sensor (respectively 7636RLU +/- 5932; n=5 and 3100RLU +/- 1610; n=3). Another background signal could be considered as the residual luciferase expression obtained after maximal inhibition with high (but not toxic) concentrations (100 µM) of the anti-NS5B cVVS-023477 78947RLU +/- 3071; n=7 which is far below the uninhibited signal (115,271 RLU +/- 60,800; n=22). The different background signals were rather equivalent and the calculated Signal/Background reached 16 for minimum of 3 required.

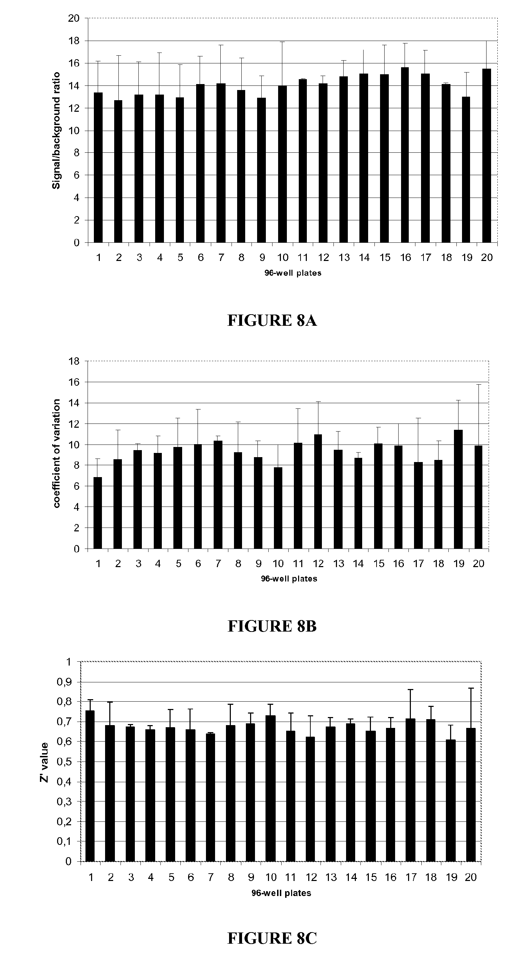

### Figures 8A, 8B and 8C: Statistical analysis of mock HTS runs

Hela cells were transfected with the three 3D-Screen vectors then plated in twenty 96-well plates to mimic HTS runs. The cells were incubated for 24 hours with or without reference drugs.
Figure 8A: Signal (cells incubated with 0.5% DMSO) over background (residual signal after reference drug cVVS-023477 addition at 100 µM final concentration) ratio is represented for each 96-well plate in 3 independent experiments.
Figure 8B: The coefficient of variation of the inhibited signal measured for cells incubated with 0.5% DMSO is represented for each 96-well plate in 3 independent experiments.
Figure 8C: The Z' factor was calculated for each 96-well plate in 3 independent experiments. The maximal signal was measured for cells incubated with 0.5% DMSO and the minimal one measured after reference drug cVVS-023477 addition at 50 µM final concentration.

### Figures 9A and 9B: Validation of the 3D-sensors in mammalian cells for the taget NS4A-NS3 protease

Hela cells were co-transfected with plasmids expressing: (i) the 3D-sensor isolated in yeast fused to the trans-activation domain (VP16-AD); (ii) the target polymerase (NS4A-NS3 protease fused to the yeast Ga14-DBD) and (iii) the luciferase reporter gene whose expression is inducible by the complex [NS4A-NS3 protease / 3D-sensor-VP16].
Figure 9A: Luciferase activation by the 3D-Sensors isolated in yeast with the peptidomimetic drug. (N=3) Measurements of luciferase expression in the absence of any drug were represented for the sixteen 3D-sensors isolated in yeast using the reference drug cVVS-023518. Cells carrying the three plasmids showed a strong activation of the luciferase reporter for 4 of the tested peptides with a ratio signal over background greater than 23.
Figure 9B: Reactivity of the most potent 3D-Sensors to the reference peptidomimetic drug. (N=3) Percentages of luciferase inhibition in the presence of the peptidomimetic NS3 specific ligand cVVS-023518 were represented for the four 3D-sensors presenting the best luciferase activation (ratio signal over background greater than 10). The 3D-sensor V7-62 was selected for its higher luciferase activation, its better responsiveness to dissociation by a specific anti-NS3 and its least data variability.

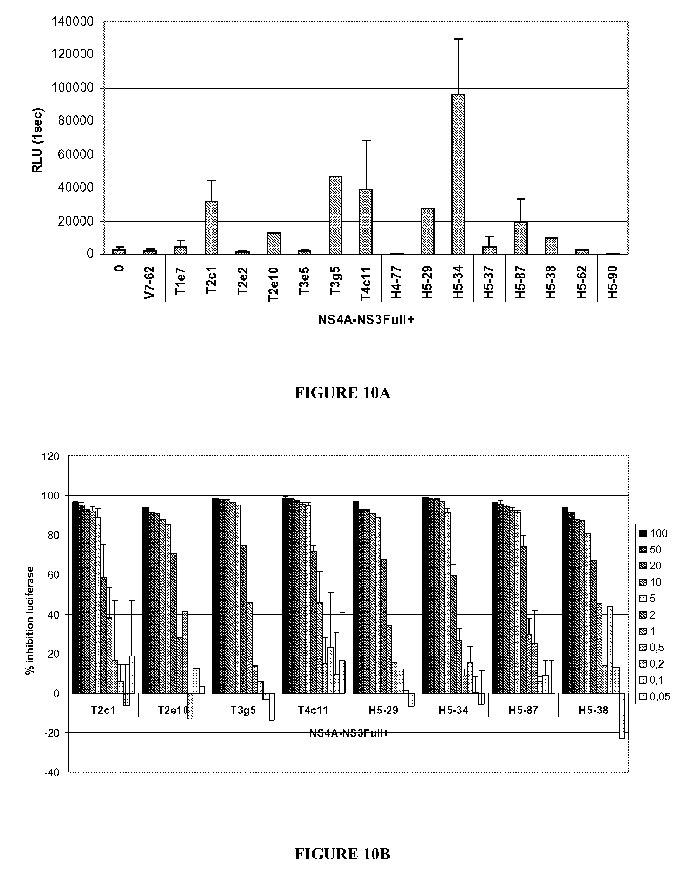

### Figures 10A and 10B: Validation of the 3D-sensors in mammalian cells for the taget NS4A-NS3 full length

Hela cells were co-transfected with plasmids expressing: (i) the 3D-sensor isolated in yeast fused to the trans-activation domain (VP16-AD); (ii) the target polymerase (NS4A-NS3 full length fused to the yeast Ga14-DBD) and (iii) the luciferase reporter gene whose expression is inducible by the complex [NS4A-NS3 full length / 3D-sensor-VP16].
Figure 10A: Luciferase activation by the 3D-Sensors isolated in yeast with the peptidomimetic drug. (N=2) Measurements of luciferase expression in the absence of any drug were represented for the sixteen 3D-sensors isolated in yeast using the reference drug cVVS-023518. Cells carrying the three plasmids showed a strong activation of the luciferase reporter for 8 of the tested peptides with a ratio signal over background greater than 16.
Figure 10B: Reactivity of the most potent 3D-Sensors to the reference peptidomimetic drug. (N=2) Percentages of luciferase inhibition in the presence of the peptidomimetic NS3 specific ligand cVVS-023518 were represented for the four 3D-sensors presenting the best luciferase activation (ratio signal over background greater than 10). The 3D-sensor H5-34 was selected for its higher luciferase activation, its better responsiveness to dissociation by a specific anti-NS3 and its least data variability.

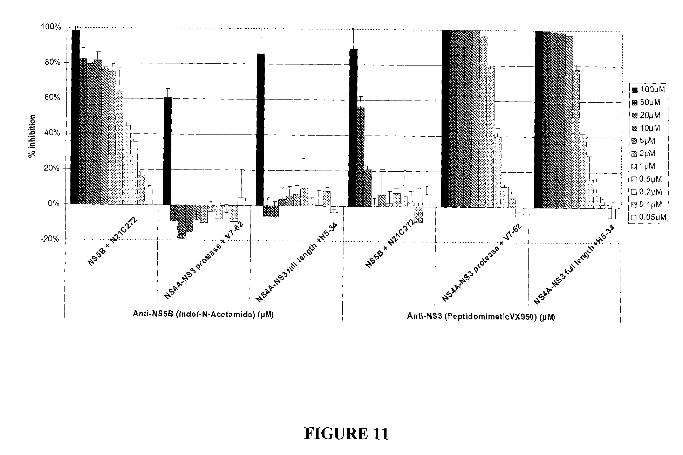

### Figure 11: Drugs' selectivity determination using the 3D-Screen platform

Hela cells were transiently transfected with the luciferase reporter and each of the NS5B or NS3 targets together with their specific 3D-Sensor. Addition of increasing concentrations of the specific NS5B inhibitor cVVS-023476 (Indol-N-Acetamide) induced a strong inhibition only on the NS5B platform. Also, the specific NS3 inhibitor cVVS-023518 inhibited the NS3 protease and full length platforms without significant off target effects.

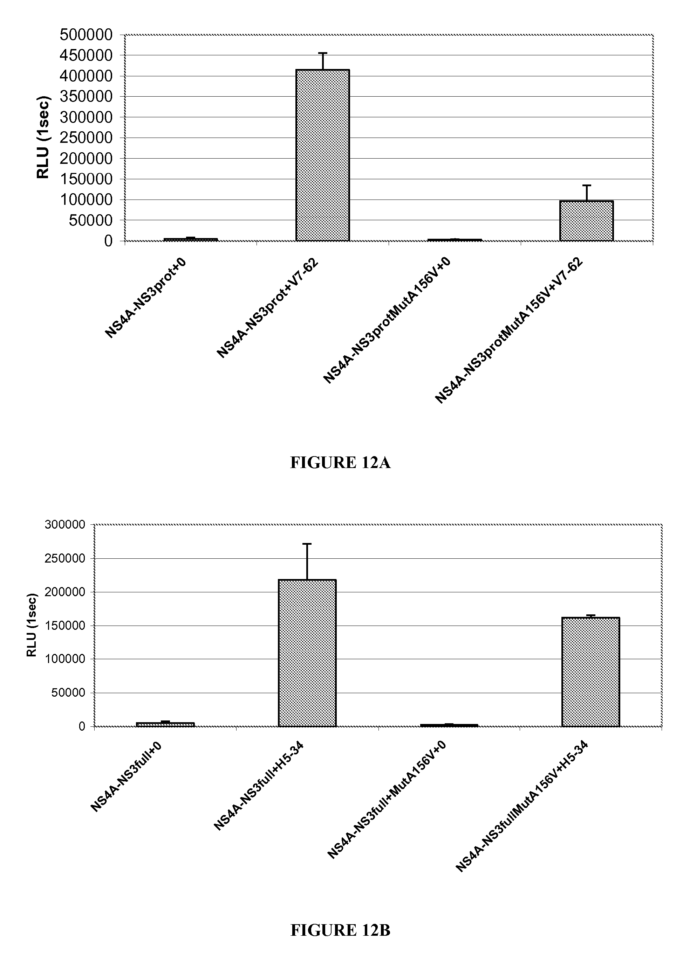

### Figures 12A and 12B: Luciferase activation by the selected 3D-Sensor in presence of a resistance mutant for NS3

Figure12A: Luciferase activation by the selected 3D-Sensor in presence of a resistance mutant for NS4A-NS3 protease (N=2). The interaction between the 3D-sensor V7-62 and either the native or A156V mutated NS4A-NS3 protease led to a strong activation of the luciferase reporter even if it is lower for the mutated form (33 times the background luciferase activity without the 3Dsensor-VP16AD versus 80 times for the native one).
Figure 12B: Luciferase activation by the selected 3D-Sensor in presence of a resistance mutant for NS4A-NS3 full length (N=2) The interaction between the 3D-Sensor H5-34 and either the native or A156V mutated NS4A-NS3full length protein led to a strong activation of the luciferase reporter (42 times the background luciferase activity without the 3D-Sensor-VP16AD for the native protein versus 56 times for the mutated one).

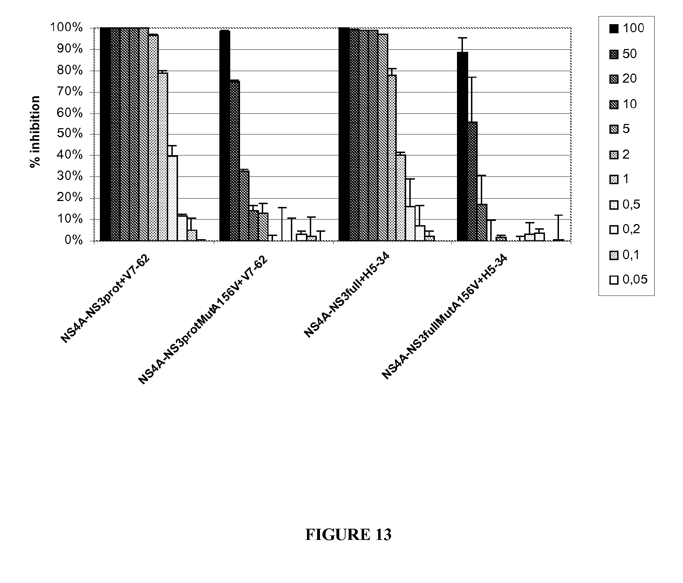

### Figure 13: Mutant 3D-Screen platform's reactivity to the peptidomimetic reference drug cVVS023517

Hela cells were transiently transfected with the luciferase reporter, the selected 3D-sensors and vectors expressing either native NS3 or a resistance mutant fused in frame with the Gal4 DNA-binding domain. Percentages of reporter's inhibition were represented for cells incubated with a specific anti-NS3 at increasing concentrations for 24 hours. The peptidomimetic compound yielded a stong reporter inhibition for the native NS4A-NS3 protease and full length while the same compound didn't show any noticeable reporter inhibition for cells transfected with the resistant mutant A156V.

### EXAMPLES

### EXAMPLE 1: Materials

### 1.1 - Chemicals:

Estradiol, Ribavirin, Phosphonoacetic acid and Foscarnet were purchased from Sigma-Aldrich (Lyon, France). The compound cVVS-023477 (N-1-cyclobutyl-4-hydroxyquinolon-3-yl-benzothiadiazin) was synthesized as described respectively in Pratt JK (2005). The compound cVVS-023476 (1-{[6-Carboxy-2-(4-chlorophenyl)-3-cyclohexyl-1H-indol-1-yl-]acetyl}-N,N-diethylpiperidin-4-aminium Chloride) was synthesized as described respectively in Harper S (2005). The compound cVVS-023518 (2-(2-{2-Cyclohexyl-2-[(pyrazine-2-carbonyl)-amino]-acetylamino}-3,3-dimethyl-butyryl)-octahydro-cyclopenta[c]pyrrole-1-carboxylic acid (1-cyclopropylaminooxalyl-butyl)-amide) was synthesized as described respectively in WO 02/183A2 (Babine R, 2002). X-Gal (5-bromo-4-chloro-3-indolyl-β-D-galactopyranoside) was purchased from Sigma-Aldrich (Lyon, France).

### Amino-acid sequence of the conformation sensitive peptides (3D-sensors) depicted and used in the following examples

| Target HCV protein | 3D-sensor peptide (Name, peptide sequence, SEQ ID number) |
|---|---|
| NS5B peptide | N21 C272 DGCARCVASVQLYGD (SEQ ID N° 59) |
| NS3 protease peptide | V7-62 WRPYYTVLCALASWH (SEQ ID N° 60) |
| NS3 full length peptide | H5-34 PSNHRQSTRSTPWLW (SEQ ID N° 61) |

### 1.2 - Buffers:

Lysis buffer: Tris Phosphate pH 7.8 (25mM) + EDTA (2mM) + DTT (1mM) + Glycerol (10%) + Triton (1%).

Luciferin buffer: Tris Phosphate pH 7.8 (20 mM) + DTT (500 mM) + MgCl2 (1.07 mM) + MgSO4 (2.7 mM) + EDTA (0.1 mM) + ATP (0.53 mM) + Acetyl coEnzyme A (0.27 mM) + Luciferin (0.47 mM).

All components were purchased from Sigma-Aldrich (Lyon, France) except Luciferin that was purchased from Promega (Charbonnières, France).

X-Gal solution: 10 ml of 0,5 M Na2HPO4 + NaH2PO4 + 50 µl of 20% SDS + 100 µl of 2% X-Gal (in DMF).

### 1.3 - Oligonucleotides for PCR were obtained from Invitrogen (Cergy Pontoise, France).

### 1.4 - Enzymes:

Restriction and ligation enzymes were purchased from Promega (Charbonnières, France) and used according to the manufacturer's recommendations. PCRs were performed with Uptitherm polymerase according the instructions of the manufacturer (Interchim, Montluçon, France).

### 1.5 - Yeast strain and culture conditions:

The *Saccharomyces cerevisiae* yeast strain L40 (MATa; his3-Δ200; leu2-3,112; trp1-901; [plexAop]4::HIS3; [plexAop]8::lacZ)) (ATCC, Molsheim, France) was cultured in either liquid (with vigorous shaking) or solid YPD or SD media supplemented with amino acids at 30°C. YPD media was composed as follows: Bacto-yeast extract 1%, Bacto-peptone 2%, Glucose 2% with or without Bacto-agar 2% (purchased from Difco). SD media was composed as follows: Yeast nitrogen base without amino acids 0.67%, Glucose 2% with or without Bacto-agar 2% (purchased from Difco, Paris, France). Amino acids were purchased from Sigma-Aldrich (Lyon, France) and added as follows: Adenine sulphate (20 mg/l SD), L-Tryptophan (50 mg/l SD), L-Histidine (50 mg/l SD, L-Leucin, L-Isoleucin, L-Valin( 60 mg/l SD for each).

### EXAMPLE 2: Recombinant plasmids

### 2.1 - Yeast expression plasmids

**pVVS468** was obtained by insertion of the genotype 1b HCV NS5BD21's sequence (GenBank D89872) between XhoI and PstI restriction sites of the polylinker of pBTM116 (Bartel & Fields, 1995) in order to be in frame with LexA (Genbank U89960). Expression of the fusion protein is under the control of the ADH1 promoter. Genotype 1b HCV NS5BD21's sequence was obtained by PCR amplification of pIV294 plasmid (kind gift Dr Catherine Schuster, Strasbourg France) carrying HCV's NS5B full-length of genotype 1b. The following oligonucleotide primers were used:
Forward primer: 5' TATCTCGAGTCAATGTCCTACACATGGACAGGTG 3' (SEQ ID N° 3). Reverse primer: 5' AGATCTGCAGTTAACGGGGTCGGGCACGAG 3' (SEQ ID N° 4).
PCR amplification was carried out for 3 min at 96°C followed by 35 cycles (30s at 94°C, 30s at 55°C and 2 min at 72°C) using a T1 thermocycler (Biometra).
**pVVS601** was obtained by insertion of the genotype 1b HCV NS3's sequence (GenBank D89872) between XhoI and BamHI restriction sites of the polylinker of pBTM116 (Bartel & Fields, 1995) in order to be in frame with LexA (Genbank U89960). Expression of the fusion protein is under the control of the ADH1 promoter. Genotype 1b HCV NS3's sequence was obtained by PCR amplification of pIV294 plasmid (kind gift Dr Catherine Schuster, Strasbourg France) carrying HCV's NS3 protease of genotype 1b. In fact, the NS3 protease has been shown to be complexed to NS4A (after cis cleavage between the two proteins) for optimal conformation and activity (Lin et al., 1995). A single chain recombinant NS4A-NS3 protease domain has shown full proteolytic activity (Taremi et al., 1998). The following oligonucleotide primers were used in order to obtain a single chain construct NS4A (residues 21-32), the GSGS linker and the NS3 protease (residues 3-181):

### Forward primer:

5'TACTCGAGGGTTCTGTTGTTATTGTTGGTAGAATTATTTTATCTGGTAGTGGTAGT ATCACGGCCTATTCCCAACAAACGCGGG 3' (SEQ ID N° 5)

### Reverse primer:

5'ACCTCGAGCAGCGCCTATCACGGCCTATTCCC 3' (SEQ ID N° 6)

PCR amplification was carried out for 3 min at 96°C followed by 35 cycles (30s at 94°C, 30s at 55° and 2 min at 72°C) using a T1 thermocycler (Biometra).

**pVVS 775** was obtained by insertion of the genotype 1b HCV NS3 full length's sequence (GenBank D89872) between XhoI and BglII restriction sites of the polylinker of pBTM116 (Bartel, 1995) in order to be in frame with LexA (Genbank U89960). Expression of the fusion protein is under the control of the ADH1 promoter. Genotype 1b HCV NS3's sequence was obtained by PCR amplification of pIV294 plasmid (kind gift of Dr Catherine Schuster, Strasbourg, France) carrying HCV's NS3 full-length of genotype 1b. Since the NS3 protease has been shown to be complexed to NS4A for optimal conformation and activity (Lin et al., 1995), a single chain recombinant NS4A-NS3 full length protein has shown full proteolytic and helicase activity (Howe et al., 1999). Moreover, it has been shown that the crystal structure of such recombinant single chain protein was superposed to the native complex NS3-NS4A (Yao et al., 1999). The following oligonucleotide primers were used in order to obtain a single chain construct NS4A (residues 21-32), the GSGS linker and the NS3 full length (residues3-631):

### Forward primer:

5'TACTCGAGGGTTCTGTTGTTATTGTTGGTAGAATTATTTTATCTGGTAGTGGTAGT ATCACGGCCTATTCCCAACAAACGCGGG 3' (SEQ ID N° 7)

### Reverse primer:

5' ATAGATCTTTTAAGTGACGACCTCCAGG 3' (SEQ ID N° 8)

PCR amplification was carried out for 3 min at 96°C followed by 35 cycles (30s at 94°C, 30s at 55° and 2 min at 72°C) using a T1 thermocycler (Biometra).
**pVVS625,** was derived from pVP16 (Hollenberg et al., 1995) by digestion with Sfi1 and insertion of a random 15 amino acids peptide library generated by PCR.

### 2.2 - Mammalian expression plasmids

**pVVS578** was derived from plasmid pUASLuc (Thiel et al., 2000) and includes a reporter construct composed of 5 Gal4-binding sites and the minimal promoter of the human -βGlobin gene followed by the firefly luciferase coding sequence.
**pVVS478** was derived from pG4mpolyII (Webster N J G, 1989) by digestion of the polylinker with Xho and BamH1 and insertion of the genotype 1b HCV's NS5B-Delta21 sequence in order to be in frame with the carboxy-terminus of the yeast Gal4 DBD (GenBank AY647987). Expression of the fusion protein is under the control of the constitutive SV40 early promoter. NS5B-delta 21 insert was obtained by PCR amplification of pIV294 plasmid using the following oligonucleotide primers: Forward primer: 5' TTC CTC GAG GAT CAA TGT CCT ACA CAT GG 3' (SEQ ID N° 9) Reverse primer: 5' GCT CGG ATC CCA GCT CTC AAC GGG GTC GGG 3' (SEQ ID N° 10). PCR amplification was carried out for 3 min at 96°C followed by 35 cycles (30s at 94°C, 30s at 55°C and 2 min at 72°C) using a T1 thermocycler (Biometra). Single mutations in the NS5B-delta21 sequence were introduced using QuickChange II XL Site-Directed mutagenesis kit according to the manufacturer's instructions (Stratagene, Amsterdam, The Netherlands).

The following primers were used to generate the plasmid **pVVS474:**

Forward primer for L30S mutation: 5' AATCCGTTGAGCAACTCTTCGCTGCGTCACCACAGTATG 3' (SEQ ID N° 11)

Reverse primer for L30S mutation: 5' ATACTGTGGTGACGCAGCGAAGAGTTGCTCAACGGATT 3' (SEQ ID N° 12)

The following primers were used to generate the plasmid **pVVS475:**

Forward primer for L30R mutation: 5' AATCCGTTGAGCAACTCTCGGCTGCGTCACCACAGTATG 3' (SEQ ID N° 13)

Reverse primer for L30R mutation: 5' CATACTGTGGTGACGCAGCCGAGAGTTGCTCAACGGATT 3' (SEQ ID N° 14)

The following primers were used to generate the plasmid **pVVS517:**

Forward primer for H95R mutation: 5' CTGACGCCCCCACGCTCGGCCAAATCC 3' (SEQ ID N° 15)

Reverse primer for H95R mutation: 5' CTGACGCCCCCACGCTCGGCCAAATCC 3' (SEQ ID N° 16)

The following primers were used to generate the plasmid **pVVS516:**

Forward primer for M414T mutation: 5' GGCAATATCATCACCTATGCGCCCACC 3' (SEQ ID N° 17)

Reverse primer for M414T mutation: 5' GGCAATATCATCACCTATGCGCCCACC 3' (SEQ ID N° 18)

The following primers were used to generate the plasmid **pVVS789:**

Forward primer for G152E mutation: 5'GGCAATATCATCACCTATGCGCCCACC 3' (SEQ ID N° 19)

Reverse primer for G152E mutation: 5'GGCAATATCATCACCTATGCGCCCACC 3' (SEQ ID N° 20)

The following primers were used to generate the plasmid **pVVS795:**

Forward primer for M423T mutation: 5' CTATGGGCGAGGACGATTCTGATGACT 3' (SEQ ID N° 21)

Reverse primer for M423T mutation: 5' AGTCATCAGAATCGTCCTCGCCCATAG 3' (SEQ ID N° 22)

The following primers were used to generate the plasmid **pWS788:**

Forward primer for N142T mutation: 5' ATCATGGCAAAAACTGAGGTTTTCTG 3' (SEQ ID N° 23)

Reverse primer for N142T mutation: 5' CAGAAAACCTCAGTTTTTGCCATGAT 3' (SEQ ID N° 24)

The following primers were used to generate the plasmid **pVVS790:**

Forward primer for P156L mutation: 5' GAGGCCGCAAGCTAGCTCGCCTTATCG 3' (SEQ ID N° 25)

Reverse primer for P156L mutation: 5' CGATAAGGCGAGCTAGCTTGCGGCCTC 3' (SEQ ID N° 26)

The following primers were used to generate the plasmid **pVVS797:**

Forward primer for P495L mutation: 5' GGAAACTTGGGGTACTACCCTTGCGAG 3' (SEQ ID N° 27)

Reverse primer for P495L mutation: 5' CTCGCAAGGGTAGTACCCCAAGTTTCC 3' (SEQ ID N° 28)

The following primers were used to generate the plasmid **pVVS791:**

Forward primer for S282T mutation: 5' GGTGCCGCGCGACCGGCGTGCTGA 3' (SEQ ID N° 29)

Reverse primer for S282T mutation: 5' TCAGCACGCCGGTCGCGCGGCACC 3' (SEQ ID N° 30)

The following primers were used to generate the plasmid **pVVS799:**

Forward primer for S368A mutation: 5' GTCATGCTCCGCCAATGTGTCG 3' (SEQ ID N° 31)

Reverse primer for S368A mutation: 5' CGACACATTGGCGGAGCATGAC 3' (SEQ ID N° 32)

The following primers were used to generate the plasmid **pVVS794:**

Forward primer for S419M mutation: 5' ATGCGCCCACCATGTGGGCGAGGATG 3' (SEQ ID N° 33)

Reverse primer for S419M mutation: 5' CATCCTCGCCCACATGGTGGGCGCAT 3' (SEQ ID N° 34)

The following primers were used to generate the plasmid **pVVS787:**

Forward primer for S96T mutation: 5' ACGCCCCCACATACGGCCAAATCCAAA 3' (SEQ ID N° 35)

Reverse primer for S96T mutation: 5' TTTGGATTTGGCCGTATGTGGGGGCGT 3' (SEQ ID N° 36)

The following primers were used to generate the plasmid **pVVS796:**

Forward primer for Y448M mutation: 5' GTAGCAGGCCCCCATGATCTGACA 3' (SEQ ID N° 37)

Reverse primer for Y448M mutation: 5' GTAGCAGGCCCCCATGATCTGACA 3' (SEQ ID N° 38)

**pVVS602** was derived from pG4mpolyII (Webster N J G, 1989) by digestion of the polylinker with XhoI and BamH1 and insertion of the genotype 1b HCV NS3's sequence in order to be in frame with the carboxy-terminus of the yeast Gal4 DBD (GenBank AY647987). Expression of the fusion protein is under the control of the constitutive SV40 early promoter. A single chain constructed NS4A (residues 21-32), the GSGS linker and the NS3 protease (residues 3-181) (Taremi et al., 1998) was obtained by PCR amplification of pIV294 plasmid using the following oligonucleotide primers:

Forward primer:

5’TACTCGAGCAGGTTCTGTTGTTATTGTTGGTAGAATTATTTATCTGGTAGTGGTA
GTATCACGGCCTATTCCCAACAAACGCGGG 3’ (SEQ ID N° 39)

Reverse primer: 5' ACCTCGAGCAGCGCCTATCACGGCCTATTCCC 3' (SEQ ID N° 40)

PCR amplification was carried out for 3 min at 96°C followed by 35 cycles (30s at 94°C, 30s at 55°C and 2 min at 72°C) using a T1 thermocycler (Biometra).

Single mutations in the NS4A-NS3 protease sequence were introduced using QuickChange II XL Site-Directed mutagenesis kit according to the manufacturer's instructions (Stratagene).

The following primers were used to generate the plasmid **pVVS781:**

Forward primer for A156V mutation: 5' GGCATCTTCCGGGTTGCTGTGTG 3' (SEQ ID N° 41)

Reverse primer for A156V mutation: 5' CACACAGCAACCCGGAAGATGCC 3' (SEQ ID N° 42) **pVVS772** was derived from pG4mpolyII (Webster N J G, 1989) by digestion of the polylinker with Xho and BamH1 and insertion of the genotype 1b HCV's NS3 full length sequence (GenBank D89872) in order to be in frame with the carboxy-terminus of the yeast Gal4 DBD (GenBank AY647987 ). Expression of the fusion protein is under the control of the constitutive SV40 early promoter. A single chain constructs NS4A (residues 21-32), the GSGS linker and the NS3 protease (residues 3-631) (Howe et al., 1999) was obtained by PCR amplification of pIV294 plasmid using the following oligonucleotide primers:

Forward primer:

5’TACTCGAGCAGGTTCTGTTGTTATTGTTGGTAGAATTATTTATCTGGTAGTGGTA
GTATCACGGCCTATTCCCAACAAACGCGGG 3’ (SEQ ID N° 43)

Reverse primer: 5' ATAGATCTTTTAAGTGACGACCTCCAGG 3' (SEQ ID N° 44)

PCR amplification was carried out for 3 min at 96°C followed by 35 cycles (30s at 94°C, 30s at 55°C and 2 min at 72°C) using a T1 thermocycler (Biometra).

Single mutations in the NS4A-NS3 full length sequence were introduced using QuickChange II XL Site-Directed mutagenesis kit according to the manufacturer's instructions (Stratagene, Amsterdam, The Netherlands).

The following primers were used to generate the plasmid **pVVS783:**

Forward primer for A156V mutation: 5' GGCATCTTCCGGGTTGCTGTGTG 3' (SEQ ID N° 45) Reverse primer for A156V mutation: 5' CACACAGCAACCCGGAAGATGCC 3' (SEQ ID N° 46)
**pVVS527** was derived from pCINéo (Promega) in order to be the recipient of the selected peptide sequences in frame with frame with the carboxy-terminus of the viral VP16 activation domain (GenBank AF294982).
**pVVS550** was derived from the pVVS527 by Sfi I digestion and insertion of the selected peptideN21C272 coding sequence. Expression of the fusion protein is under the control of the constitutive CMV promoter.

The insert was obtained by PCR amplification using the following oligonucleotide primers:

Forward primer: 5' TCCTTAAGGGAGATGTGAGCAT 3' (SEQ ID N° 47)

Reverse primer: 5' CAAGGCGATTAAGTTGGTAAC 3' (SEQ ID N° 48)

PCR amplification was carried out for 3 min at 96°C followed by 35 cycles (1 min at 94°C, 1 min at 60°C and 1 min at 72°C) using a T1 thermocycler (Biometra).

### EXAMPLE 3: Identification of peptides interacting with the bait

The naïve yeast was transformed with the pVVS468 (NS5BD21 fused to LexA) according to the Lithium Acetate transformation protocol (Schiestl *et al*, 1989). Transformants were grown in liquid culture under selective pressure (SD medium without Tryptophan), before being transformed with pVVS625 (library of random peptides in frame with the viral VP16 activation domain). Resulting double transformants were spread on SD Agar medium lacking Tryptophan and Leucine, and incubated for 48 hours at 30°C. Colonies were then scraped and mixed. 1/500^{th} of the suspension obtained was plated on SD Agar medium lacking Tryptophan, Leucine and Histidine to identify HIS3 reporter's activation. After 3 to 5 days of growth, an X-Gal overlay was performed to identify LacZ activation. An X-Gal solution was mixed with an equal volume of 1% agarose cooled to 50°C then poured slowly over yeast colonies on the surface of agar plates. The individual clones that stained blue were picked up to be grown overnight in deep wells in order to be further analyzed.

### 3.1 - Identification of 3D-sensors

Yeast colonies containing a peptide interacting with the bait were grown overnight in 96-well deepwells in trp-leu- liquid medium. They were then spotted using a plugger either on trp-leu-his-or trp-leu- solid medium supplemented with different concentrations of specific or non specific drugs or an equivalent amount of solvent (DMSO). Yeast colonies loosing expression of reporter genes were selected. Result was confirmed at least 3 times.

Another method used was a semi-quantitative β-galactosidase liquid assay. Yeast colonies were grown as described above in liquid selective medium with or without the reference drug at different concentrations. The cell pellet was resuspended in 100 ul of Y-PER protein extraction reagent (Pierce, Brebières, France). After 30 mn of shaking at 300 rpm, the supernatant was clarified and the total protein concentration determined using the Micro BCA^{™} Protein Assay Reagent Kit from Pierce according to the manufacturer's instructions. 5 ul of the protein extract were used to determine Lac Z activity by adding 180u1 of ONPG (O-nitrophenyl-β-D-galactopyranoside) solution at 0.66 mg/ml (Sigma -Aldrich, Lyon, France)). The reaction was stopped as soon as a yellow coloration has developed by adding 75 ul of 1 molar solution of Na₂CO₃. OD was measured at 420 nm on a spectrophotometer and the specific activity was calculated using the following formula:

Specific activity (nmol/mg/min) = OD420X Vt / (0.0045 X Protein X Ve X Time)
where Vt is the total volume reaction (160 ul), Protein is the protein concentration of the yeast extract in mg/ml, Ve is the extract volume assayed in ml, and Time is the time of reaction in minutes.

### 3.2 - Plasmid rescue

For plasmid extraction, yeast clones whose reporter expression was inhibited by specific drugs were grown in trp-leu- liquid medium. 2 ml of overnight saturated culture were spinned down for 5 min in a microfuge and the pellet was resuspended in 80 ul of lysis buffer supplemented with 20 µl of 2500 Units/ml of Lyticase (Sigma-Aldrich, Lyon, France). The suspension was incubated 1 h at 37°C with shaking at 220 rpm. 20 µl of 20% SDS were added and the mix was vortexed for 1 min. The mixture was then purified using the Nucleospin Plasmid Quickpure according to the manufacturer's instructions (Macherey Nagel, Hoerdt, France). PCR was performed on the purified eluate using the following primers:

Forward primer: 5' TCCTTAAGGGAGATGTGAGCAT 3' (SEQ ID N° 49)

Reverse primer: 5' CAAGGCGATTAAGTTGGTAAC 3' (SEQ ID N° 50)

PCR products were sequenced and cloned in the pVVS527 vector as described above. Plasmids were purified and verified by enzymatic restriction and DNA sequencing.

### 3.3 - Cell lines and culture conditions

Hela cells (ATCC, Molsheim, France) were maintained in EMEM (Minimum Essential Medium Eagle with Earle's Balanced Salt solution without L-Glutamine; Biowhittaker/Cambrex, East Rutherford, New Jersey, USA) supplemented with 10% FBS (JRH), 2 mM Glutamine (Biowhittaker/Cambrex), 1% Non-essential Amino acids (Biowhittaker/Cambrex), 100 units/ml Penicillin and 100 ug/ml Streptomycin. Cells were kept in a humidified 5% CO₂ atmosphere at 37°C and dissociated using Trypsine-EDTA (1X) when reaching 80-90% confluence.

### 3.4 - Hela cells' transfection experiments

Transfections were performed for 2 hours in 100 mm dishes in 4 million cells with 5 ug of total DNA complexed to jetPEI (PolyPLus transfection, Illkirch, France) with a N/P ratio of 8 The optimal ratio between the plasmids encoding for the luciferase reporter, the target and the selected 3Dsensor was: 3ug/1ug/0.5ug. Cells were dissociated and 25000 cells per well were seeded in 96 well culture treated plates (BD Biosciences, Erembodegem, Belgium).

### 3.5 - Small molecule library screening

Compound library's plates were thawed at room temperature. Drugs were handled by a Genesis workstation (Tecan, Lyon, France) and added to the cells at a 5 uM (in 0.5% DMSO) final concentrations. Screening was performed by runs of twenty plates. Hits were identified by measurement of the luciferase activity.

### 3.6 - Luciferase assay

Cell culture supernatant was discarded and 100 ul of Lysis buffer were added to each well. Following 10 mn of vigorous shaking, the lysate was transferred to white opaque 96 well-plates and 100u1 of Luciferin buffer were added. Chemi-luminescence measurement was performed immediately using a Microlumat LB96 luminometer (Berthold, Thoiry, France) with 1 or 10 sec integration time.

### 3.7 - Cytotoxicity assay

Viability measurements were performed on cells transfected and seeded in 96 well plates as described above and incubated with drugs for 24 hours. 0.15mg/ml of XTT solution (Roche Diagnostics, Meylan, France) were added to each well and the plates incubated for 1 hour at 37°C. The optical density of each well's cell culture supernatant was analysed using a spectrophotometer (at 450 nm with deduction of background signal at 690 nm).

### 3.8 - IC50 determination experiments

Selected hits were synthesized and tested at concentrations varying between 0.01 uM and 100 uM final concentration so as to determine their IC50 on 3 independent experiments.

### EXAMPLE 4: The 3D-Screen-NS5B platform

### 4.1 - Identification of conformation sensitive peptides interacting with NS5B-Delta21 in yeast

Yeast two-hybrid experiments were carried in the L40 strain (ATCC, Molsheim, France) transformed with a lexA/truncated HCV polymerase lacking the C-terminal 21 amino-acids fusion protein as a bait and the VVS001 peptide library as a prey. The C-terminal 21 amino-acid domain anchors the protein to the cell membrane which compromises the feasibility of a two-hybrid approach (Yamashita et al., 1998; Dimitriva et al., 2003). Removal of the 21 amino-acids domain does not compromise the NS5B activity and introduces minor changes in the overall protein conformation (Yamashita et al., 1998; Toméi et al., 2000). A total of 576 colonies were selected for their ability to grow under selective conditions (Figure 1a). Conformation sensitive peptides or "3D-sensors" were then identified by addition to the selected individual yeast colonies of cVVS-023477 or cVVS-023476, two anti-NS5B drugs known to induce conformational changes in the target polymerase (figure1b). Growth and LacZ expression of 16 colonies were abolished upon addition of cVVS-023477, a compound that belongs to the family of Benzothiadiazins (Pratt et al., 2005). Another 22 colonies were also identified with the cVVS-023476, an Indol-N-Acetamid compound (Harper et al., 2005) using a semi quantitative approach (data not shown).

Plasmids encoding for the selected peptides were isolated and used to confirm in yeast the ability of each 3D-sensor to bind to the target as well as to study specificities of interaction.

Figure 1c shows the growth and lac Z expression ofN21C272, the most sensitive peptide, in absence of Histidine and in presence of either cVVS-023477 (Benzothiadiazidic anti-NS5B) or non specific viral polymerase ligands. Foscarnet, an anti-CMV/HIV polymerases (Crumpacker et al., 1992; Lietman et al., 1992) and its analog, phosphonoacetic acid (PAA) (Crumpacker et al., 1992), had no effect on this selected clone. Estradiol was also used as a negative control while it was active on the 3Dsensor N° 30.2-ER interaction reported previously by our group (See WO 2006/046134) (that is not affected by the anti-NS5B cited above).

None of the peptides' sequences exhibited any significant homology with known proteins (A BLAST search of the National Center for Biotechnology Information database). All sequences were inserted in frame with VP16 AD in expression vectors to perform the screening of small molecules in mammalian cells.

### 4.2 - Validation of the 3D-sensors in human cells

3D-sensors confirmed in yeast were further investigated in human cells. Among the reasons of choosing Hela cells figured their wide use, easiness of manipulation and capacity of replicating subgenomic HCV replicons (Zhu et al., 2003; Kato et al., 2005). The cells were co-transfected with plasmids expressing: (i) the 3D sensor peptide candidate fused to a trans-activation domain (VP16-AD); (ii) the target polymerase(NS5BD21 fused to the yeast Ga14-DNA Binding Domain) and (iii) the luciferase reporter gene whose expression is inducible by the complex [NS5BD21/3D-sensor-VP16]. 6 of the selected 3D-sensors supported activation of the luciferase reporter gene in the absence of any ligand (figure 2A). This result confirms the interaction of those peptides with the NS5BD21 in a mammalian context. As expected, luciferase activity was strongly reduced upon addition of the Benzothiadiazidin cVVS-023477 (figure 2B). The 3D-sensor N21C272 was selected to generate the 3D-Screen platform on the basis of (i) signal intensity, (ii) inhibition of the signal in the presence of cVVS-023477 (51% luciferase inhibition at 1uM and 90% at 10uM final concentration). Figures 2C and 2D represent luciferase expression and inhibition by specific and non specific polymerase inhibitors for the 3D-sensors identified using cVVS-023476.

### 4.3 - Development of the NS5B-3D-Screen platform

### 4.3.1) Selectivity of the NS5B-Delta21 3D-sensor peptide

To further characterize the 3D-Screen platform, the selectivity of binding of peptide N21C272 was investigated. Hela cells were transiently transfected with the luciferase reporter, the 3D-sensor N21C272 and vectors expressing either the native NS5BD21 or different mutants (figure 3A). The interaction between the native NS5BD21 and the 3D-sensor N21C272 led to a strong activation of the luciferase reporter which represents 17 times the background luciferase activity without the 3Dsensor-VP16AD. However, the 3D-sensor did not bind the L30S and L30A conformational destabilizing mutants described by Labonté P (2002).

On the other hand, as shown in figure 3B, the 3D-sensor N21C272 did bind to 10 of the 12 drug-resistant mutants tested: the mutants described as resistant to Benzothiadiazins (M414T, S266A and C316Y) (Toméi et al., 2005; Lu et al., 2007), the Indol resistant mutant P495L (Toméi et al., 2005), the Tiophens resistant mutants (S419M and M423T) (Toméi et al., 2005), the 2' nucleoside analogues resistant mutants S282T, S96T and N142T (Toméi et al., 2005; Le Pogam et al., 2006) and the G152E mutant resistant to Dihydroxypyrimidines (Toméi et al., 2005).

### 4.3.2) Drugs' specificity of action on the 3D-Screen platform

The inventors next confirmed that dissociation of the 3D-Sensor from the polymerase resulted from binding of relevant drugs. Hela cells were transiently transfected with the luciferase reporter, the 3D-sensor N21C272/VP16 and the fusion protein NS5BD21-Gal4. Addition of the specific NS5B inhibitors cVVS-023477 and cVVS-023476 induced a strong inhibition of the luciferase signal at 10 uM final concentration (respectively 83.2% +/- 7.5; n=15 and 82.6 % +/-8.3; n=6) as shown on figure 4. cVVS-023476 showed potent inhibition of the luciferase signal even at lower concentrations (67% +/- 8.3 at 1 uM final concentration, n=6). In contrast, the reporter's expression was not inhibited by addition of anti-CMV/HIV polymerase inhibitors such as Foscarnet or Phosphonoacetic acid (Crumpacker et al., 1992; Lietman et al., 1992) at 10 uM concentration (respectively 11.7% +/- 4 and -2.2%+/-33.8; n=3). The mechanism of action of Ribavirin that represents (in combination with Interferon) the standard HCV treatment, does not seem to involve a direct inhibition of the polymerase (Lohmann et al., 2000; Maag et al., 2001). Accordingly, used at 75 uM final concentration (its EC50), Ribavirin didn't show any noticeable effect on the luciferase signal (inhibition 8.7% +/-16.3; n=6). We thus confirmed that, like in yeast, the 3D-sensor peptide was able, in human cells, to interact in a selective way with the polymerase, but not with the polymerase complexed with specific allosteric anti-NS5B ligands.

### 4.3.3) Concentration-response relationship

To validate whether drug's action on our system was stokiometric or not, Hela cells were transiently transfected with three expression plasmids encoding the reporter gene luciferase, the target protein and the conformation sensitive peptide N21C272. After incubation with increasing amounts of reference drugs cVVS-023476 or cVVS-023477, luciferase expression was measured. Figure 5 shows that a concentration-effect relationship according to the Michaelis-Menten model was obtained. The IC50 values for cVVS-023476 and cVVS-023477 were respectively of 0.3 and 3.2 uM.

### 4.3.4) Mutant profiling

Since the specificity of drugs' action on the 3D-Screen platform could be useful for screening of molecules active on resistant mutants, the inventors transfected Hela cells with the luciferase reporter, the 3D-sensorN21C272 and vectors expressing either native NS5B-Delta21 or different mutants described in the literature. After incubation for 24 hours with increasing concentrations of cVVS023477 (a compound belonging to the Benzothiadiazin family) or cVVS-023476 (a compound belonging to the Indol N Acetamid family), luciferase expression was measured. Figure 6A shows that the benzothiadiazidic compound yielded a comparable reporter inhibition for either the native NS5BD21 or the P495L, the S423M or the S282T mutants sensitive to Benzothiadiazins with an IC50 between 2 and 5 uM (n=2). On the other hand, the same compound did not show any noticeable reporter inhibition for cells transfected with the mutants described as resistant to Benzothiadiazins. In fact, for the M414T and C316Y mutants, the 50% signal inhibition was not reached and for the S368A, the IC50 is ten folds the one observed on the native NS5B. When we tested the Indol N Acetamid compound, the inventors noticed that it selectively inhibited the native NS5BD21 and all tested mutants (IC50 between 0.2 and 2 uM) except the P495L one described as resistant to Indols with an increase of IC50 of at least 10 fold (figure 6B, n=2).

These results show that one could generate a cellular platform that is selective to the target's conformation as far as it is not disturbed by destabilising mutations. Furthermore, the resistance mutants platform of the invention allows to discriminative between active and inactive drugs according to the target's resistance profile.

### 4.3.5) Compatibility of the NS5BD21/N21C272- 3D-Screen platform with High throughput screening

The assay was further optimized to meet high throughput requirements starting with the signal window. Figure 7 shows that the residual signal after maximal luciferase inhibition was as low as the platform's background (8947RLU+/-3071 for cVVS023477; n=7 versus 7182RLU+/- 6643 for basal luciferase activity without 3Dsensor; n=17). The signal emitted by cells transfected with the reporter, the 3D-sensor and either the L30S or the L30R NS5B denaturated mutants was also equivalent to the platform's background (7636 RLU+/-5932 for the L30S mutant; n=5) and 3100 RLU+/-1610 for the L30R one; n=3).

In order to evaluate the assay's variability, twenty 96-well plates containing cells transfected with the plasmids encoding for the luciferase reporter, NS5BD21 and the 3D sensor N21C272 were plated in the presence of 0.5% DMSO for 24 hours in 3 independent experiments. Calculated S/B reached 14 +/- 0.9 (figure 8A) and CV 9.3%+/-1 (figure 8B). Mean Z' values of 0.67 +/- 0.03 (figure 8C) were obtained indicating low variability over a large dynamic range.

### 4.4 - High throughput screening of a library of test compounds

### 4.4.1 - Primary screening

The 23510 original compounds issued from VIVALIS proprietary combinatorial library (i.e the Vivatheque) were screened in HeLa cells. The three expression plasmids: luciferase reporter, NS5B-Delta21-Gal4 and 3D-sensor/VP16AD (N21C272) were co-transfected in 4 million Hela cells in 10 cm dishes. After 2 hours, cells were dissociated and 25,000 cells/well were seeded in 96-well plates. Chemical compounds were added after cell adhesion. Luciferase activity was measured 24 H later. 1437 hits yielding 40% of luciferase inhibition were identified during the primary screening (hit rate 6.1 %).

### 4.4.2 - Secondary screening

342 hits (1.45% of total screened compounds) were confirmed on a second and third round of transfections which represents a confirmation rate of 23.8%. Among those, 215 compounds showed a cytotoxicity less than 40 %.

### 4.4.3 - IC50 determination experiments

102 of the selected compounds were purchased as pure powders so as to determine their 50% inhibitory concentration. 29 showed interesting dose response profiles independently from cytotoxicity. For 18 molecules, the IC50 value was below 10 uM with a toxicity less than 20% at that concentration. The viral inhibitory potential of 8 of them was confirmed using both the reporter replicon system in Huh7(5,2) and the RT-PCR quantitative replicon system in Huh7(9,13). Three of the molecules identified by our platform were previously patented for their anti-HCV properties. The first one, cVVS 013005 (aminothiazol) was reported in the patent WO 2006/122011, the second one cVVS019534 (thiosemicarbazone) was described in the patent WO 2004/060308 and the third one a thienopyridine was described in the patent WO 2006/019832.

### EXAMPLE 5: The 3D-Screen-NS3 platform

### 5.1 - Identification of conformation sensitive peptides

### 5.1.1) Identification of conformation sensitive peptides interacting with NS4A-NS3 protease in yeast

Yeast two-hybrid experiments were carried in the L40/yDG1 strain transformed with a lexA/HCV NS4A-NS3 protease domain (amino-acids 1-181) fusion protein as a bait and the WS001 peptide library as a prey. The inventors screened 864 interacting peptides in presence or not of a reference drug cVVS023518 (a peptidomimetic inhibitor in phase 2 of clinical trial (at 50 uM concentration) (Perni et al., 2006). 23 of them were selected for their signal extinction in presence of the reference drug using a quantitative Beta Galactosidase liquid assay.

Plasmids encoding for the selected peptides were isolated and used to confirm in yeast the ability of each 3D-sensor to bind to the target as well as to study specificities of interaction. Amino acid sequences of the 3D-sensor peptides identified with the cVVS023518 were determined. None of the peptides' sequences exhibited any significant homology with known proteins (BLAST search of the National Center for Biotechnology Information database). All sequences were inserted in frame with VP16 AD in expression vectors to perform the screening of small molecules in mammalian cells.

### 5.1.2) Identification of conformation sensitive peptides interacting with NS4A-NS3 full length protein in yeast

Yeast two-hybrid experiments were carried in the L40 strain transformed with a lexA/HCV NS4A-NS3 full length (amino-acids 1-632) fusion protein as a bait and the WS001 peptide library as a prey. We screened 768 interacting peptides in presence or not of a reference drug cVVS023518 (a peptidomimetic inhibitor in phase 2 of clinical trial (at 50 uM concentration) (Perni et al., 2006). 15 of them were selected for their reactivity to the reference drug using a quantitative Beta Galactosidase liquid assay. Plasmids encoding for the selected peptides were isolated and used to confirm in yeast the ability of each 3D-sensor to bind to the target as well as to study specificities of interaction.

Amino acid sequences of the 3D-sensor peptides identified with the cVVS023518 were determined. None of the peptides' sequences exhibited any significant homology with known proteins (BLAST search of the National Center for Biotechnology Information database. All sequences were inserted in frame with VP16 AD in expression vectors to perform the screening of small molecules in mammalian cells.

### 5.2 - Validation of the 3D-sensors in human cells

### 5.2.1) Validation of the 3D-sensors interacting with NS4A-NS3protease

3D-sensors confirmed in yeast were further investigated in Hela cells. The cells were co-transfected with plasmids expressing: (i) the 3D sensor peptide candidate fused to a transactivation domain (VP16-AD); (ii) the target NS4A-NS3 protease fused to the yeast Gal4-DNA Binding Domain; and (iii) the luciferase reporter gene whose expression is inducible by the complex [NS4A-NS3protease/3D-sensor-VP16]. 4 of the 23 tested 3D-sensors supported activation of the luciferase reporter gene in the absence of any ligand (figure 9A). This result confirms the interaction of those peptides with the NS4A-NS3 protease in a mammalian context. As expected, luciferase activity was strongly reduced upon addition of the peptidomimetic inhibitor cVVS-023518 (figure 9B).

The 3D-sensor V7-62 was selected to generate the 3D-Screen platform on the basis of (i) signal intensity, (ii) inhibition of the signal in the presence of cVVS-023518 (90% luciferase inhibition at 2 uM final concentration).

### 5.2.2) Validation of the 3D-sensors interacting with NS4A- NS3full length protein

3D-sensors confirmed in yeast were further investigated in Hela cells. The cells were co-transfected with plasmids expressing: (i) the 3D sensor peptide candidate fused to a transactivation domain (VP16-AD); (ii) the target NS4A- NS3full length protein fused to the yeast Gal4-DNA Binding Domain; and (iii) the luciferase reporter gene whose expression is inducible by the complex [NS4A- NS3full length protein /3D-sensor-VP16]. 8 of the 16 identified 3D-sensors supported activation of the luciferase reporter gene in the absence of any ligand (figure 10A). This result confirms the interaction of those peptides with the NS4A- NS3full length protein in a mammalian context. As expected, luciferase activity was strongly reduced upon addition of the peptido-mimetic inhibitor cVVS-023518 (figure 10B). The 3D-sensor H5-34 was selected to generate the 3D-Screen platform on the basis of (i) signal intensity, (ii) inhibition of the signal in the presence of cVVS-023518 (60% luciferase inhibition at 2 uM final concentration).

### 5.3 - Drugs' selectivity determination using the 3D-Screen platform

The inventors confirmed that dissociation of the 3D-Sensor from the NS3 target resulted from binding of relevant drugs and not due to specific events. Hela cells were transiently transfected with the luciferase reporter and each of the NS5B and NS3 targets together with their specific 3D-Sensor. Addition of increasing concentrations of the specific NS5B inhibitor cVVS-023476 (Indol-N-Acetamide) induced a strong inhibition of only the NS5B platform. Also, the specific NS3 inhibitor cVVS-023518 inhibited the NS4A-NS3protease and full length platforms without significant off target effects (Figure 11). These results demonstrate that the screening platform of the invention could be used to test the selectivity of drugs on different targets in a cellular context.

### 5.4 - Mutant profiling NS4A-NS3 protease 3D-Screen platform

Since the specificity of drugs' action on the 3D-Screen platform could be useful for screening of molecules active on resistant mutants, the inventors transfected Hela cells with the luciferase reporter, the 3D-sensor V7-62 and vectors expressing either native NS4A-NS3 protease or one of the most frequently described mutants resistant to VX950 described in the literature (Lin et al., 2005). The interaction between the 3D-sensor V7-62 and either the native or A156V mutated NS4A-NS3 protease led to a strong activation of the luciferase reporter even if it is lower for the mutated form (33 times the background luciferase activity without the 3D sensor-VP16AD versus 80 times for the native one) (figure 12A). After incubation for 24 hours with increasing concentrations of cVVS-023518, luciferase expression was measured. Figure 13 shows that the peptido-mimetic compound yielded a strong reporter inhibition for the native NS4A-NS3 protease while the same compound didn't show any noticeable reporter inhibition for cells transfected with the resistant mutant A156V.

### 5.5 - Mutant profiling NS4A- NS3full length protein 3D-Screen platform

The inventors transfected Hela cells with the luciferase reporter, the 3D-sensor H5-34 and vectors expressing either native NS4A-NS3full length protein or one of the most frequently described mutants resistant to the peptidomimetic described in the literature (Lin et al., 2005). The interaction between the 3D-sensor H5-34 and either the native or A156V mutated NS4A-NS3full length protein led to a strong activation of the luciferase reporter (42 times the background luciferase activity without the 3D sensor-VP16AD for the native protein versus 56 times for the mutated one) (figure 12B). After incubation for 24 hours with increasing concentrations of cVVS-023518, luciferase expression was measured. Figure 13 shows that the peptidomimetic compound yielded a stong reporter inhibition for the native NS4A- NS3 full length protein while the same compound did not show any noticeable reporter inhibition for cells transfected with the resistant mutant A156V.

These results show that one could generate a cellular platform that is selective of the target's conformation as far as it is not disturbed by destabilising mutations. Furthermore, the resistance mutants platform of the invention could discriminate active and inactive drugs according to the target's resistance profile.

### REFERENCES

Amin A., J. Zaccardi, S. Mullen, S. Olland, M. Orlowski, B. Feld, P. Labonte, and P. Mak. 2003. Identification of constrained peptides that bind to and preferentially inhibit the activity of the hepatitis C viral RNA-dependent RNA polymerase. Virology. 313:158-169.
**Babine RE et al., 2002.** Peptidomimetic protease inhibitors. WO 02/18369 A2.
**Barsanti P. et al. 2004.** Thiosemicarbazone as anti-HCV. WO2004060308.
Bartel PL, Fields S. 1995. Analyzing protein-protein interactions using the two-hybrid system. Methods Enzymol., Vol. 254:241-263.
Bartenschlager R. 2002. Hepatitis C virus replicons: potential role for drug development. Nat Rev Drug Discov 1:911-6
Beaulieu P.L. et al. 2007, Current Opinion in investigational Drugs ; Vol.8(8), 621
Behrens S-E, L. Tomei, and R De Francesco.1996. Identification and properties of the RNA-dependent RNA polymerase of hepatitis C virus. The EMBO Journal. Vol. 15 N° 1:12-22.
Beske, O. E., and S. Goldbard. 2002. High-throughput cell analysis using multiplexed array technologies. Drug Discov Today 7:5131-5.
Bourne N., R. B. Pyles, M. Yi, R. L. Veselenak, M. M. Davis, S. M. Lemon. 2005. Screening for hepatitis activity with a cell-based secreted alkaline phosphatase reporter replicon system. Antiviral Reasearch. 67:76-82.
Choo QL et al., 1989. Isolation of a cDNA clone derived from a blood-borne non-A, non-B viral hepatitis genome. Science, 244:359-62.
Crumpacker CS 1992. Mechanism of action of foscarnet against viral polymerases. Am J Med Vol. 14 N° 2A:3S-7S.
De Clercq E. et al., 2007, Nature Review, Drug Discovery, 6, 1001-1018.
De Francesco R.D. et al., 2007, Advanced Drug Delivery Reviews,59, 1242-1262.
Dimitrova M., I Imbert, M.P. Kieny, and C. Schuster. 2003. Protein-Protein Interactions between Hepatitis C Virus Nonstructural Proteins. Journal of Virology. 77: 5401-14.
**Fowlkes D et al.** 2002. Methods of identifying conformation-sensitive binding peptides and uses thereof. WO 02/004956
Fried MichaelW. 2002. Side effects of therapy of hepatitis and their management. Hepatology Vol. 32:S237-244.
Gordon, C. P. and P. A. Keller. 2005. Control of Hepatitis C: A Medicinal Chemistry Perspective. Journal of Medicinal Chemistry, 48: 1-20.
Harper S., B. Pacini, S. Avolio, M. Di Filippo, G. Migliaccio, R. laufer, R De Francesco, M Rowley, and F Narjes. 2005. Development and Preliminary Optimization of Indole-N-Acetamide Inhibitors of Hepatitis C Virus NS5B Polymerase. J. MED. Chem; 48:1314-1317.
Harper S., S. Avolio, B. Pacini, M. Di Filippo, S. Altamura, L. Tomei, G. Paonessa, S. Di Marco, A. Carfi, C. Giuliano, J. Padron, F. Bonelli, G. Migliaccio, R. De Francesco, R. Laufer, M. Rowley, and F. Narjes. 2005. Potent Inhibitors of Subgenomic Hepatitis C Virus RNA Replication through Optimization of Indole-N-Acetamide Allosteric Inhibitors of the Viral NS5B Polymerase. Journal of Medicinal Chemistry. Vol. 48, N° 14:4547-57.
Hao W et al., 2006. Development of a novel dicistronic reporter-selectable hepatitis C virus replicon suitable for high throughput inhibitor screening. Antimicrob. Agents Chemother., 51:95-102.
Hollenberg SM., R. Sternglanz, P. F. Cheng, and H. Weintraub. 1995. Identification of a New Family of Tissue-Specific Basic Helix-Loop-Helix proteins with a Two-Hybrid System. Molecular and Cellular Biology. Vol. 15 N° 7:3813-22.
Howe AY, Chase R, Taremi SS, Risano C, Beyer B, Malcolm B, Lau JY. 1999. A novel recombinant single-chain hepatitis C virus NS3-NS4A protein with improved helicase activity. Protein Sci., Jun;8(6):132-41.
Hugle, T., and A. Cerny. 2003. Current therapy and new molecular approaches toantiviral treatment and prevention of hepatitis C. Rev Med Virol. 13:361-71.
**Karp G, Chen G. 2006.** Thienopyridines for treating Hepatitis C. WO 2006/019832.
Kato T., T. Date, M Miyamoto, M. Mizokami, and T. Wakita. 2005. Non hepatic cell lines Hela and 293 support efficient replication for the Hepatitis C virus genotype 2a subgenomic replicon. J. Virol. Vol. 79 N° 1:592-596.
Krieger, N., V. Lohmann, and R. Bartenschlager. 2001. Enhancement ofhepatitis C virus RNA replication by cell culture-adaptive mutations. J. Virol. 75:4614-24.
Labonté P., V. Axelrod, A. Agarwal, A. Aulabaugh, A. Amin, and P. Mak. 2002. Modulation of Hepatitis C Virus RNA-dependent RNA Polymerase Activity by Structure-based Site-directed Mutagenesis. The Journal of Biological Chemistry. Vol. 277, N° 41:38838-46.
Lake-Bakaar, G. 2003. Current and future therapy for chronic hepatitis C virusliver disease. Curr Drug Targets Infect Disord 3:247-53.
Lamarre D. et al., 2003. An NS3 protease inhibitor with antiviral effects in humans infected with hepatitis C virus. Nature. Vol 426: 186-189.
LePogam L et al. 2006. Selection and characterization of replicon variants dually resistant to thumb and palm binding nonnucleoside polymerase inhibitors of the hepatitis C virus. Journal of Virology. Vol. 80 N° 12:6146-6154.
Lindenbach, B. D., M. J. Evans, A. J. Syder, B. Wolk, T. L. Tellinghuisen, C.C. Liu, T. Maruyama, R. O. Hynes, D. R. Burton, J. A. McKeating, and C. M. Rice. 2005. Complete Replication of Hepatitis C Virus in Cell Culture. Science 309:623-626.
Lietman PS. 1992.Clinical pharmacology: Foscarnet. Am. J. Med. Vol. 92 N° 2A:8S-11S.
Lin C et al., 1995. A Central Region in the Hepatitis C Virus NS4A Protein Allows Formation of an Active NS3-NS4A Serine Proteinase Complex In Vivo and In Vitro. Journal of virology 69:4373-4380.
Lin C et al., 2005. In vitro studies of cross resistance mutations against two hepatitis virus protease inhibitors VX950 and BILN2061. J. Biol. Chem. Vol. 280 N+44:36784-36791.
Lohmann V., F. Körner, U. Herian, and R. Bartenschlager. 1997. Biochemical Properties of Hepatitis C Virus NS5B RNA-Dependent RNA Polymerase and Identification of Amino Acid Sequence Motifs Essential for Enzymatic Activity. Journal of Virology. Vol. 71 N° 11:8416-28.
Lohmann V., F. Körner, A. Dobierzewska, and R. Bartenschlager. 2000. Mutations in Hepatitis C Virus RNAs Conferring Cell Culture Adaptation. Journal of Virology. Vol. 75, N° 3:1437-49.
Lohmann, V., F. Korner, J. Koch, U. Herian, L. Theilmann, and R. Bartenschlager. 1999. Replication of subgenomic hepatitis C virus RNAs in a hepatoma cell line. Science 285:110-3.
Lu L et al., 2007. Identification and characterization of mutations conferring resistance to an HCV RNA-dependant RNA polymerase inhibitor in vitro. Antiviral Res. Vol. 76(1):93-97. **Luo G. 2000**
Maag D., C. Castro, Z. Hong, and C. E. Cameron. 2001. Hepatitis C Virus RNA-dependent RNA Polymerase (NS5B) as a Mediator of the Antiviral Activity of Ribavirin. The Journal of Biological Chemistry. Vol. 276, N° 49:46094-98.
Manns M.P.et al., 2007, Nature Review, Drug Discovery, 6, 991-1000.
McHutchison JG. 2004. Understanding HepatitisC. Am. J. Manag. Care 10(2S):521-9.
McKercher G., P. L. Beaulieu, D. Lamarre, S. LaPlante, S Lefebvre, C. Pellerin, L. Thauvette and G. Kukolj. 2004. Specific inhibitors of HCV polymerase identified using an NS5B with lower affinity for template/primer substrate. Nucleic Acids Research. Vol. 32 N° 2:422-431.
Moussalli J, Opolon P, Poynard T. 1998.Management of hepatitis C. J. Viral. Hepatitis. Vol. 5:73-82.
Nguyen TT et al. 2003. Antimicrobial Agents and Chemotherapy. Vol. 47 N° 11:3525-3530.
O'Boyle D.R., 2nd, P. T. Nower, J. A. Lemm, L. Valera, J. H. Sun, K. Rigat,R. Colonno, and M. Gao. 2005. Development of a cell-based high-throughputspecificity screen using a hepatitis C virus-bovine viral diarrhea virus dual repliconassay. Antimicrob Agents Chemother 49:1346-53.
Oh. J-W., T. Ito and M. M.C. Lai. 1999. A Recombinant Hepatitis C Virus RNA-Dependent RNA Polymerase Capable of Copying the Full-Length Viral RNA. Journal of Virology. Vol. 73, N° 9:7694-7702.
Paige LA et al. 1999. Estrogen receptor modulators each induce distinct conformational changes in the ER alpha and ER beta. Proc Natl Acad Sci USA. Vol. 96 N° 7:3999-4004.
Perni RB et al., 2006. Preclinical profile of VX-950, a potent, selective, and orally bioavailable inhibitor of hepatitis C virus NS3-4A serine protease. Antimicrobial agents and chemotherapy, 50:899-909.
Pratt J.K., P. Donner, K.F. Mc Daniel, C.J.Maring, W.M. Kati, H. Mo, T. Middleton, Y. Liu, T Ng, Q. Xie, R. Zhang, D. Montgomery, A. Molla, D.J. Kempf and W. Kohlbrenner. 2005. Inhibitors of HCV NS5B polymerase: synthesis and structure-activity relationship of N-1-heteroalkyl-4-hydroxyquinolon-3-yl-benzothiadiazines. Bioorganic & Medicinal Chemistry Letters 15:1577-1582.
Sarrazin C. et al., 2007. Dynamic Hepatits C virus genotypic and phenotypic changes in patients treated with the protease inhibitor Telaprevir. Gastroenterology Vol. 132:1767-1777.
Schiestl RH and Gietz RD. 1989. High efficiency transformation of intact yeast cells using single stranded nucleic acids as carrier. Current Genetics. Vol. 16:339-346.
Srikanth Venkatraman et al 2006. Discovery of (1R,5S)-N-[3-Amino-1-(cyclobutylmethyl)-2,3-dioxopropyl]-3-[2(S)-[[[(1,1-dimethylethyl)amino]carbonyl]amino]-3,3-dimethyl-1-oxobutyl]-6,6-dimethyl-3-azabicyclo[3.1.0]hexan-2(S)-carboxamide (SCH 503034), a Selective, Potent,Orally Bioavailable Hepatitis C Virus NS3 Protease Inhibitor: A Potential Therapeutic Agent for the Treatment of Hepatitis C Infection. J. Med. Chem., 49, 6074-6086.
Taremi SS et al., 1998. Construction and characterization of a novel fully activated recombinant single chain hepatitis C virus protease. Protein Sci. Vol. 7 N° 10:2143-2149.
Thiel G et al., 2000. The human transcriptional repressor protein NAB1: expression and biological activity. Biochim Biophys Acta. Vol. 1493 N° 3:289-301.
Tomei L., R. L. Vitale, I Incitti, S. Serafini, S. Altamura, A. Vitelli, and R De Francesco. 2000. Biochemical characterization of hepatitis C virus RNA-dependant RNA polymerase mutant lacking the C-Terminal hydrophobic sequence. Journal of General Virology. 81:759-767.
Toméi L, AltamuraS, Paonessa G, De Francesco R, MigliaccioG. 2005. HCV antiviral resistance: the impact of in vitro studies on the development of antiviral agents targeting the viral NS5B polymerase.Antiviral chemistry and chemotherapy. Vol. 16:225-245.
Trozzi C et al., 2003. In vitro selection and characterization of Hepatitis C virus serine protease variants resistant to an active-site peptide inhibitor. Journal of Virology. Vol. 77 N° 6:3669-3679.
Wakita, T., T. Pietschmann, T. Kato, T. Date, M. Miyamoto, Z. Zhao, K.Murthy, A. Habermann, H. G. Krausslich, M. Mizokami, R. Bartenschlager and T. J. Liang. 2005. Production of infectious hepatitis C virus in tissue culture from a cloned viral genome. Nat. Med. 11:791-6.
Walker M.P. and Z. Hong. 2002. HCV RNA-dependent RNA polymerase as a target for antiviral development. Current Opinion in Pharmacology. 2:1-7.
Webster N J G, Green S, Tasset D, Ponglikimongkol M, Chambon P. 1989. The transcriptional activation function located in the hormone-binding domain of the human oestrogen receptor is not encoded in a single exon. EMBO Journal. 8(5):1441-6.
Yao N et al., 1999. Molecular views of viral polyprotein processing revealed by the crystal structure of the hepatitis C virus bifunctional protease-helicase. Research article, 7:1353-1363.
You X., A. W. Nguyen, A. Jabaiah, M. A. Sheff, K. S. Thorn, and P. S. Daugherty.2006. Intracellular protein interaction mapping with FRET hybrids. PNAS. 103:18458-463.
**Zhang S.,** 2006. Thiazole compounds and methods of use.WO 2006/122011.
Zhong, J.P. Gastaminza, G. Cheng, S. Kapadia, T. Kato, D. R. Burton, S. F. Wieland, S.L. Uprichard, T. Wakita, and F.V. Chisari. 2005. Robust hepatitisC virus infection in vitro. Proc Natl Acad Sci U S A, 102:9294-9.
Zhu Q., J-T. Guo and C Seeger. 2003. Replication of Hepatitis C Virus Subgenomes in Nonhepatic Epithelial and Mouse Hepatoma Cells. Journal of Virology. Vol. 77 N° 17:9204-10. Zuck, P., E. M. Murray, E. Stec, J. A. Grobler, A. J. Simon, B. Strulovici, J.Inglese, O. A. Flores and M. Ferrer. 2004. A cell-based beta-lactamase reportergene assay for the identification of inhibitors of hepatitis C virus replication. Anal Biochem. 334:344-55.

## Claims

1. An *in vitro* method of determining the ability of a test compound to modulate the biological activity of a variant of a target protein, wherein a ligand is previously known to modulate the biological activity of said target protein, said method comprising the steps of:
Step A) Selecting at least one binding peptide which binds to said target protein and to said variant of the target protein, said method comprising the steps of: A1) providing a combinatorial library of peptides where said binding peptide is a member of said library, wherein said library is expressed in a plurality of cells and said cells collectively expressed all members of said library; A2) screening said library for the ability of its members to bind to said target protein and to said variant of the target protein, and selecting the peptide(s) binding to said target protein and to said variant of the target protein;
Step B) selecting among the selected peptide(s) of step A2), at least one binding peptide having a decreased or no ability to bind to said target protein in presence of said known ligand and a conserved ability to bind to said variant of target protein in presence of said known ligand;
Step C) testing and selecting a test compound for its ability to decrease the binding of the peptide(s) selected in step B) to said target protein, wherein a decrease or an absence of binding ability is indicative that the test compound induces a conformational change of the target protein, indicating that said test compound modulates the biological activity of said target protein; and
Step D) testing the test compound selected in C) for its ability to modulate the binding ability of the peptide(s) selected in step B) to said variant of the target protein wherein:
- a decrease or an absence of binding is indicative that the test compound induces a conformational change to said variant of target protein, indicating that said test compound modulates the biological activity of said target protein, said variant of the target protein being not resistant to the modulation of its biological activity by said test compound;
- a conserved binding is indicative that the test compound does not induce a conformational change to said variant of target protein, indicating that said test compound does not modulate the biological activity of said target protein, said variant of target protein being resistant to the modulation of its biological activity by said test compound.

2. The method according to claim 1 wherein steps A), B), C) and D) are performed in a cell, not integrated into a whole multi-cellular organism or a tissue or organ of an organism.

3. The method according to claim 1 or 2 wherein steps A) and B) are performed in a yeast cell and steps C) and D) are performed in a mammalian cell selected among human primary cells, human embryonic cells.

4. The method according to one of claims1 to 3 wherein in step A) and B), each cell is co-expressing:
- said target protein or said variant of target proteins, or a ligand-binding protein moiety thereof, and
- one member of said combinatorial library of peptides, and each cell is further providing a signal producing system operably associated with said target protein or variant of target proteins, or moiety, such that a signal is produced which is indicative of whether said member of said library binds said target protein or moiety in or on said cell.

5. The method according to claims 1 to 4 wherein the known ligand is endogenously or preferably exogenously added to the cells of step B.

6. The method of claims 4 and 5, wherein said signal is produced when said peptide binds to said target protein and to said variant of target protein, said signal is decreased or absent when said peptide is unable to bind to said target protein or to said variant of target protein, liganded to a known ligand to said target protein.

7. The method according to claims 1 to 6 wherein steps C) and D) are performed in a cell, wherein said cell co-expressing:
a) said target protein or said variant of target protein, or a ligand-binding protein moiety thereof; and
b) said peptide selected in step B) and able to bind to the target protein and to said variant of target protein in absence of known ligand; and and wherein said cell further providing a signal producing system operably associated with said target protein or said variant of target protein, or a ligand-binding protein moiety thereof whereby:
- the binding of said peptide to said protein in presence of test compound results in the constitution of a functional transactivation activator protein which activate expression of said reporter gene, whereby a signal is produced which is indicative that said peptide binds said target protein or said variant of target protein, or moiety, in or on said cell of steps C) and D), and that said test compound does not modify the conformation of said target protein or variant of target protein; or
- the decrease or the absence of binding of said peptide to said protein in presence of the test compound does not allow the constitution of a functional transactivation activator protein, whereby no signal is produced which is indicative that said test compound modify the conformation of said target protein or variant of said target protein.

8. The method of claims 4 to 7 where said signal producing system comprises:
- a protein-bound component which is fused to said target protein or said variant of target protein, or a ligand-binding protein moiety thereof, so as to provide a chimeric protein; and
- a peptide-bound component which is fused to said peptide so as to provide a chimeric peptide, whereby a signal is produced when the peptide-bound and protein-bound components are brought into physical proximity as a result of the binding of the peptide to the target protein.

9. The method of claim 8 where one of said components is a DNA-binding domain (DBD) and another of said components is a complementary transactivation domain (AD), and the signal producing system further comprises at least one reporter gene operably linked to an operator bound by said DNA-binding domain, the binding of the peptide to the target protein resulting in the constitution of a functional transactivation activator protein which activates expression of said reporter gene.

10. The method of any claims 4 to 9 wherein said signal producing system comprises:
(i) a complementary transactivation domain (AD) which is fused to said peptide to provide a chimeric peptide; and
(ii) a DNA-binding domain (DBD) which is fused to said target protein or said variant of target protein, or a ligand-binding protein moiety thereof, to provide a chimeric protein; and
(iii) a signal producing system comprising at least a reporter gene operably linked to an operator bound by said DBD, whereby the binding of said peptide to said protein, results in the constitution of a functional transactivation activator protein which activate expression of said reporter gene, whereby a signal is produced which is indicative of the binding of said peptide to said target protein, or a variant of target-protein, or a ligand-binding protein moiety thereof, in or on said cell used in steps A), B), C) or D).

11. The method of any claims 4 to 10 wherein the DBD is selected from the group consisting of Gal4 and LexA and where the AD is selected from the group consisting of *E. coli* B42, Gal4 activation domain II, and HSV VP16.

12. The method of claims 6 to 9 where the reporter gene is a resistance selection gene selected from the group comprising yeast genes HIS3, LEU2, TRP1, URA and antibiotic resistance genes.

13. The method of claims 4 to 13 where the reporter gene used for screening and selecting the test compound in steps C) and D) is selected from the group consisting of the genes encoding by the following proteins: DHFR, luciferase, chloramphenicol acetyl-transferase, beta-lactamase, adenylate cyclase, alkaline phosphatase, and beta-galactosidase and autofluorescent proteins.

14. The method of claims 1 to 13 in which the test compound is endogenously or, preferably, exogenously added to the cell of steps C) and D).

15. The method of claims 1 to 14 where said target protein is selected among viral proteins, bacterial proteins, vegetal proteins, animal proteins and human proteins.

16. The method of claims 1 to 15 where said variant of target protein is selected among mutation containing target proteins, polymorphisms of said target proteins, proteins containing sequence homology with said target proteins.

17. The method of claims 1 to 16 wherein said target protein is a hepatitis C virus protein selected among core protein, glycoproteins E1 and E2, NS1, NS2, NS3, NS4A, NS4B, NS5A, NS5B.

18. An *in vitro* method of determining the ability of a test compound to modulate the biological activity of a variant of a hepatitis C virus protein selected among core protein, glycoproteins E1 and E2, NS1, NS2, NS3, NS4A, NS4B, NS5A, NS5B, wherein a ligand is previously known to modulate the biological activity of said target protein, said method comprising the steps of:
Step A) Selecting one binding peptide which binds to said target protein and to said variant of the target protein, said method comprising the steps of: A1) providing a combinatorial library of peptides where said binding peptide is a member of said library, wherein said library is expressed in a plurality of cells and said cells collectively expressed all members of said library; A2) screening said library for the ability of its members to bind to said target protein and to said variant of the target protein, and selecting the peptide(s) binding to said target protein and to said variant of the target protein;
Step B) selecting among the selected peptide(s) of step A2), at least one binding peptide having a decreased or no ability to bind to said target protein in presence of said known ligand and a conserved ability to bind to said variant of target protein in presence of said known ligand;
Step C) testing and selecting a test compound for its ability to decrease the binding of the peptide(s) selected in step B) to said target protein, wherein a decrease or an absence of binding ability is indicative that the test compound induces a conformational change of the target protein, indicating that said test compound modulates the biological activity of said target protein; and
Step D) testing the test compound selected in C) for its ability to modulate the binding ability of the peptide(s) selected in step B) to said variant of the target protein wherein:
- a decrease or an absence of binding is indicative that the test compound induces a conformational change to said variant of target protein, indicating that said test compound modulates the biological activity of said target protein, said variant of the target protein being not resistant to the modulation of its biological activity by said test compound;
- a conserved binding is indicative that the test compound does not induce a conformational change to said variant of target protein, indicating that said test compound does not modulate the biological activity of said target protein, said variant of target protein being resistant to the modulation of its biological activity by said test compound.

19. The method according to claim 17 or 18 wherein said target protein is hepatitis C virus protein NS5B, and wherein said variant of NS5B is preferably selected among NS5B variants selected in the group comprising M414T, S368A, C316Y, Y448H, P495L, S419M, M423T, S282T, S96T, N142T, G152E, P156L.

20. The method according to claim 19 wherein the known ligand of the hepatitis C target protein NS5B is selected among benzothiazadin (*N*-1-cyclobutyl-4-hydroxyquinolon-3-yl-benzothiadiazin), N-indol acetamid (1-{[6-Carboxy-2-(4-chlorophenyl)-3-cyclohexyl-1*H*-indol-1-yl-]acetyl}-*N,N*-diethylpiperidin-4-aminium Chloride), HCV796, GS 9190, MK3281 and VCH 759.

21. The method according to claim 18 wherein said target protein is hepatitis C virus protein NS3, and wherein said variant of NS3 is preferably selected among NS3 variants selected in the group comprising A156V/S/T, D168A/V, V107A, R155K, T54A, V36M, V36M/A156T, V36A/A156, V36M/R155K, V36T/T54, V36M/T54A, A156V/R155K.

22. The method according to claim 21 wherein the known ligand of the hepatitis C target protein NS3 is selected among peptidomimetic anti-NS3 protease (2-(2-{2-Cyclohexyl-2-[(pyrazine-2-carbonyl)-amino]-acetylamino}-3,3-dimethyl-butyryl)-octahydro-cyclopenta[c]pyrrole-1-carboxylic acid (1-cyclopropylaminooxalyl-butyl)-amide), BILN2061; ITMN 191,TMC 435350 and SCH503034.
